# EUROPEAN PATENT APPLICATION

(11) **EP 1 642 594 A1**
(43) Date of publication of application: **05.04.2006**
(21) Application number: 04746831.9
(22) Date of filing: 25.06.2004
(51) Int. Cl.: A61K 45/06, A61K 31/277, A61K 31/195, A61P 13/00, A61P 13/02, A61P 13/10, A61P 43/00

(54) **REMEDY FOR URINARY TRACT DISEASES**

(30) Priority: 27.06.2003 JP 2003185168
(71) Applicant: ONO PHARMACEUTICAL CO., LTD., Osaka-shi, Osaka 541-8526 (JP)
(72) Inventor: MARUYAMA, Takayuki, Ono Pharmaceutical Co Ltd, Mishima-gun, Osaka 618-8585 (JP); KOBAYASHI, Michiyoshi, Ono Pharmaceutical Co Ltd, Mishima-gun, Osaka 618-8585 (JP); NONAKA, Shigeyuki, Ono Pharmaceutical Co Ltd, Mishima-gun, Osaka 618-8585 (JP); OKADA, Hiroki, Ono Pharmaceutical Co Ltd, Mishima-gun, Osaka 618-8585 (JP); KONEMURA, Takashi, Ono Pharmaceutical Co Ltd, Mishima-gun, Osaka 618-8585 (JP)
(74) Representative: Henkel, Feiler & Hänzel
(86) International application number: PCT/JP2004/009362
(87) International publication number: WO 2005/000356

(57) **Abstract**

A preventive and/or a remedy for urinary tract diseases with symptoms such as urgency of urination, bladder pain, frequent urination or urine incontinence which comprises a combination of compound having antagonism to EP₁ with another compound having antagonism to EP₃ each selected from among prostaglandin E₂ receptors. The combination of an EP₁ antagonist with an EP₃ antagonist is useful in preventing and/or treating urinary tract diseases with symptoms such as urgency of urination, bladder pain, frequent urination or urine incontinence, because of showing effect of improving urine retaining ability, improving bladder compliance, relieving hypertonic detrusor muscle and normalizing bladder perception.

## Description

### TECHNICAL FIELD

The present invention relates to an agent for treating urinary tract diseases. More specifically, the present invention relates to an agent for preventing and/or treating urinary tract diseases which comprises a combination of EP₁ agonist with EP₃ agonist.

### BACKGROUND ART

The urinary tract disease is state that there is a trouble somewhere of the route to which urine is excreted, and it can be divided roughly into urinary storage disorder and voiding disorder. Typical symptoms of voiding disorder include dysuria, constant urge to urinate and anuresis. Typical symptoms of urinary storage disorder include urgency of urination, bladder pain, frequent urination, night urination and urine incontinence. The cause of the symptom of urinary storage disorder include decreasing the storage capacity of the bladder, decreasing bladder compliance, hypertonic detrusor muscle, involuntary contraction, bladder afferent hypersensitivity and urinary sphincter dysfunction. There are neurogenic bladder, nervous pollakisuria, cystitis, urethritis, prostatitis, prostatic hypertrophy, bladder tumor or prostatic cancer *etc*. as diseases that cause urinary storage disorder. Incontinence includes urgency incontinence, stress urinary incontinence, overflow incontinence, psychogenic incontinence or complex incontinence etc.

At present, a medicine that decreases contraction of detrusor muscle as anticholinergic drugs is used in the treatment of frequent urination or urine incontinence. However, because anticholinergic drugs repress contraction of detrusor muscle necessary for urination, an increase in the amount of the residual urine is a problem. Moreover dry mouth is as side effect.

Prostaglandin (PG) E₂ has been known as a metabolite in the arachidonate cascade. It has been known that PGE₂ possesses cyto-protective activity, uterine contractive activity, a pain-inducing effect, a promoting effect on digestive peristalsis, an awakening effect, a suppressive effect on gastric acid secretion, hypotensive activity and diuretic activity and so on.

A recent study has proved existence of various PGE₂ subtype receptors possessing a different physiological or pharmacological role from each other. At present, four receptor subtypes are known and they are called EP₁, EP₂, EP₃, and EP₄ (Negishi M., *et al., J. Lipid Mediators Cell Sigtialing,* 12, 379-391 (1995)).

Among these subtypes, it is known that EP₁ receptor relates to pain, fever or diuresis. It is known that EP₃ receptor relates to signal transduction of peripheral nerve, control of exothermal reaction in central nerve, formation of memory by expressing in cerebral neuron, vascularization, reabsorption of urine by expressing in renal tubular, uterine contraction, production ofACTH, platelet aggregation.

However, at present, concrete diseases for which the compounds having antagonism to EP₁ receptor and the compounds having antagonism to EP₃ receptor are used have not been established. It has not been established to use those combinations for the disease treatment. Moreover, concrete diseases for which the combinations of them are used have not been established.

The compounds having antagonism to EP₁ receptor include, for example, compounds described in WO98/27053, compounds described in EP878465 or compounds described in WO02/72564. Moreover, the agent for treating frequent urination and incontinence of urine are is indicated in the specifications of WO03/43655 and WO01/19819, respectively.

The compounds having antagonism to EP₃ receptor include, for example, compounds described in WO02/16311 and compounds described in WO02/20462. It is known that compounds described in WO03/16254 have antagonism to EP₃ receptor and EP₄ receptor. Moreover, compounds having antagonism to EP₄ receptor are described in WO01/62708.

The compounds having antagonism to EP₃ receptor include, for example, compounds described in WO02/16311 and compounds described in WO02/20462. It is known that compounds described in WO03/16254 have antagonism to EP₃ receptor and EP₄ receptor. Moreover, compounds having antagonism to EP₄ receptor are described in WO01/62708.

### DISCLOSURE OF THE INVENTION

Since there are various causes of the urinary tract disease and treatments therefor are not uniform, the medicines being used now are not satisfactory and have problems such as side effects.

The present inventors have energetically studied to find a novel agent for treating urinary tract diseases with strong effect and without side effect. As a result, they found out that the medicament comprising the combination of EP₁ agonist and EP₃ agonist achieves the purpose unexpectedly.

The present invention relates to the followings.
1. An agent for preventing and/or treating urinary tract disease comprising a combination of EP₁ antagonist and EP₃ antagonist.
2. The agent for preventing and/or treating urinary tract disease according to 1 above, wherein the urinary tract disease is lower urinary tract disorder.
3. The agent for preventing and/or treating urinary tract disease according to 1 above, wherein the urinary tract disease is urinary storage disorder.
4. The agent for preventing and/or treating urinary tract disease according to 3 above, wherein the urinary storage disorder is overactive bladder.
5. The agent for preventing and/or treating urinary tract disease according to 4 above, wherein the overactive bladder is urgency of urination, bladder pain or urine incontinence.
6. The agent for preventing and/or treating urinary tract disease according to 4 above, wherein the overactive bladder is frequent urination.
7. The agent for preventing and/or treating urinary tract disease according to 5 above, wherein the urine incontinence is urgency incontinence, stress urinary incontinence, overflow incontinence, psychogenic incontinence or complex incontinence.
8. The agent for preventing and/or treating urinary tract disease according to 1 above, which is an agent for improving urine retaining ability.
9. The agent for preventing and/or treating urinary tract disease according to 1 above, which is an agent for improving bladder compliance.
10. The agent for preventing and/or treating urinary tract disease according to 1 above, which is an agent for relieving hypertonic detrusor muscle.
11. The agent for preventing and/or treating urinary tract disease according to 1 above, wherein the EP₁ antagonist is a compound selected from a group consisting of
   a compound represented by formula (A) 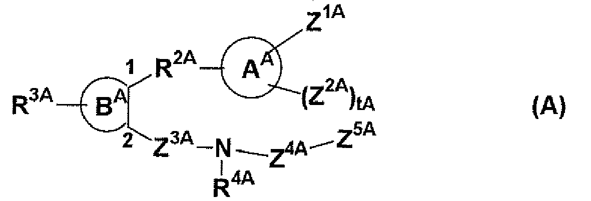 wherein 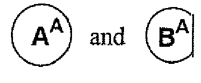 are each independently C5-15 carbocyclic ring or 5-to 7-membered heterocyclic ring having 1 or 2 oxygen, sulfur or nitrogen atoms;
   Z^{1A} is a group represented by -COR^{1A}, -C1-4 alkylene-COR^{1A}, -CH=CH-COR^{1A}, -C≡C-COR^{1A}, -O-C1-3 alkylene-COR^{1A} wherein R^{1A} is hydroxy, C1-4 alkoxy or a group represented by formula NR^{6A}R^{7A} wherein R^{6A} and R^{7A} are independently hydrogen atom or C1-4 alkyl or -C1-5 alkylene-OH;
   Z^{2A} is a hydrogen atom, C1-4 alkyl, C1-4 alkoxy, nitro, halogen, trifluoromethyl, trifluoromethoxy, hydorxy or a group represented by formula COR^{1A} wherein R^{1A} has the same meaning as described above;
   Z^{3A} is a single bond or C1-4 alkylene;
   Z^{4A} is SO₂ or CO;
   Z^{5A} is (1) C1-8 alkyl, C2-8 alkenyl, C2-8 alkynyl, (2) phenyl, C3-7 cycloalkyl, 5-to 7-membered heterocyclic ring having 1 or 2 oxygen, sulfur or nitrogen atoms, (3) C1-4 alkyl, C2-4 alkenyl or C2-4 alkynyl substituted by phenyl or C3-7 cycloalkyl wherein phenyl, C3-7 cycloalkyl and 5- to 7-membered heterocyclic ring having 1 or 2 oxygen, sulfur or nitrogen atoms in above-described (2) and (3) may by substituted by 1 to 5 R^{5A} groups wherein multiple R^{5A}'s are independently a hydrogen atom, C1-6 alkyl, C1-6 alkoxy, C1-6 alkylthio, nitro, halogen, trifluoromethyl, trifluoromethoxy or hydroxy;
   R^{2A} is CONR^{8A}, NR^{8A}CO, CONR^{8A}-C1-4 alkylene, C1-4 alkylene-CONR^{8A}, NR^{8A}CO-C1-4 alkylene, C1-4 alkylene-NR^{8A}CO, C1-3 alkylene-CONR^{8A}-C1-3 alkylene, C1-3 alkylene-NR^{8A}CO-C1-3 alkylene wherein R^{8A} is a hydrogen atom or C1-4 alkyl, O, S, NZ^{6A} wherein Z^{6A} is a hydrogen atom or C1-4 alkyl, Z^{7A}-C1-4 alkylene, C1-4 alkylene-Z^{7A}, C1-3 alkylene-Z^{7A}-C1-3 alkylene wherein Z^{7A} is O, S or NZ^{6A} wherein Z^{6A} has the same meaning as described above, CO, CO-C1-4 alkylene, C1-4 alkylene-CO, C1-3 alkylene-CO-C1-3 alkylene, C2-4 alkylene, C2-4 alkenylene or C2-4 alkynylene;
   R^{3A} is a hydrogen atom, C1-6 alkyl, C1-6 alkoxy, C1-6 alkylthio, nitoro, halogen, trifluromethyl, trifluoromethoxy, hydroxy or hydroxymethyl;
   R^{4A} is (1) a hydrogen atom, (2) C1-8 alkyl, C2-8 alkenyl, C2-8 alkynyl, (3) C1-6 alkyl substituted by 1 or 2 group(s) selected from COOZ^{8A}, CONZ^{9A}Z^{10A}, OZ^{8A} wherein Z^{8A}, Z^{9A} and Z^{10A} are independently a hydrogen atom or C1-4 alkyl, C1-4 alkoxy-C1-4 alkoxy, (4) C3-7 cycloalkyl, (5) C1-4 alkyl, C2-4 alkenyl or C2-4 alkynyl substituted by phenyl or C3-7 cycloalkyl wherein phenyl or C3-7 cycloalkyl in above-described (4) and (5) may be substituted by 1 to 5 R^{5A} groups wherein R^{5A} has the same meaning as described above; n^{A} and t^{A} are each independently an integer from 1 to 4, and
   wherein
   (1) R^{2A} and Z^{3A} are each connected at the 1- or 2-position of 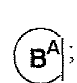 and
   (2) when 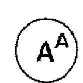 is benzene and (Z^{2A})_{tA} is other than COR^{1A}, Z^{1A} is connected at the 3- or 4-position of benzene,
   a salt thereof, a solvate thereof or a prodrug thereof,
   a compound represented by formula (B) 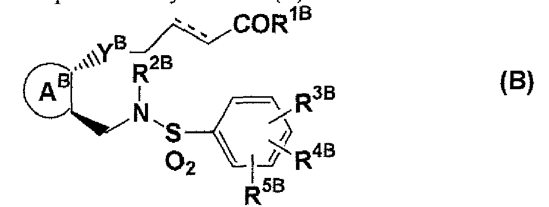 wherein 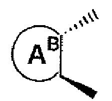 is a group represented by formula 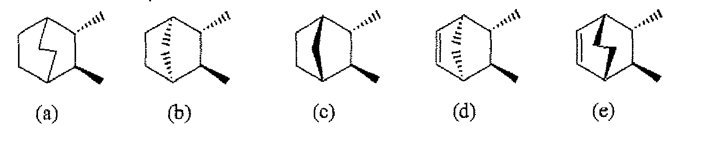 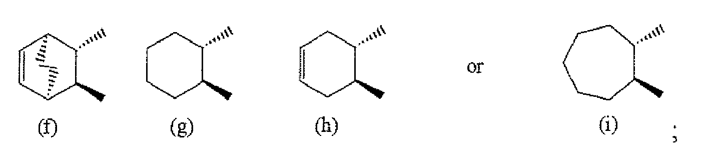 R^{1B} is hydroxy, C1-4 alkoxy or a group represented by formula NR^{6B}R^{7B} wherein R^{6B} and R^{7B} are each independently a hydrogen atom or C1-4 alkyl;
   R^{2B} is a hydrogen atom or C1-4 alkyl;
   R^{3B} and R^{4B} are each C1-4 alkyl, a halogen atom or trifluoromethyl;
   R^{5B} is a hydrogen atom, C1-4 alkyl, a halogen atom or trifluoromethyl;
   Y^{B} is cis-vinylene or trans-vinylene;
   symbol 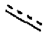 is a single bond or double bond, and
   wherein, when 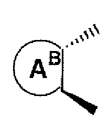 is formula 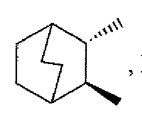 R^{1B} is hydroxy or C1-4 alkoxy, R^{2B} is a hydrogen atom, Y^{B} is cis-vinylene and symbol 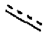 is a single bond, 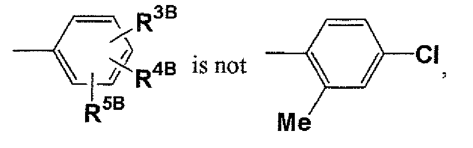 a salt thereof, a solvate thereof or a prodrug thereof, and
   a compound represented by formula (C) 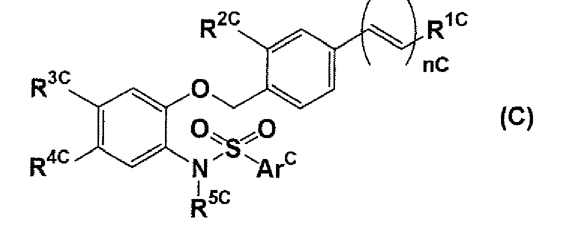 wherein R^{1C} is COOH, 5-tetrazolyl, 5-oxo-1,2,4-oxadiazolyl, CH₂OH or 5-oxo-1,2,4-thiadiazolyl;
   R^{2C} is hydrogen, methyl, methoxy or chloro;
   R^{3C} and R^{4C} are a combination of (1) methyl and methyl, (2) methyl and chloro, (3) chloro and methyl or (4) trifluoromethyl and hydrogen, or are taken together with the carbon atom to which they are attached to form (5) cyclopentene, (6) cyclohexene or (7) benzene;
   R^{5C} is isopropyl, isobutyl, 2-methyl-2-propenyl, cyclopropylmethyl, methyl, ethyl, propyl, 2-propenyl or 2-hydroxy-2-methylpropyl;
   Ar^{C} is thiazolyl which may be substituted by methyl, pyridyl or 5-methyl-2-furyl;
   n^{C} is 0 or 1, and
   wherein, when R^{1C} is 5-tetrazolyl, 5-oxo-1,2,4-oxadiazolyl or 5-oxo-1,2,4-thiadiazolyl, n^{C} is 0,
   an alkyl ester thereof, a salt thereof or a prodrug thereof.
12. The agent for preventing and/or treating urinary tract disease according to 11 above, wherein the compound is 4-[6-[N-isobutyl-N-(4-methyl-2-thiazolylsulfonyl)amino]indan-5-yloxymethyl]benzoic acid or 3-methyl-4-[6-[N-isobutyl-N-(4-methyl-2-thiazolylsulfonyl)]amino]indan-5-yloxymethyl]cinnamic acid.
13. The agent for preventing and/or treating urinary tract disease according to 1 above, wherein the EP₃ antagonist is a compound selected from a group consisting of
   a compound represented by formula (D) 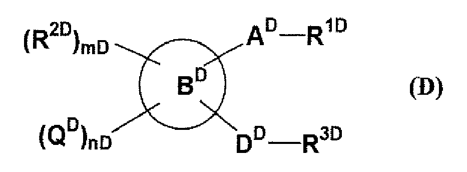 wherein R^{1D} is -COOH, -COOR^{4D}, -CH₂OH, -CONR^{5D}SO₂R^{6D}, -CONR^{7D}R^{8D}, -CH₂NR^{5D}SO₂R^{6D}, -CH₂NR^{9D}COR^{10D}, -CH₂NR^{9D}CONR^{5D}SO₂R^{6D}, -CH₂SO₂NR^{9D}COR^{10D}, -CH₂OCONR^{5D}SO₂R^{6D}, tetrazole, 1,2,4-oxadiazol-5-one, 1,2,4-oxadiazole-5-thione, 1,2,4-thiadiazol-5-one, 1,3-thiazolidine-2,4-dione, or 1,2,3,5-oxathiadiazol-2-one;
   R^{4D} is C1-6 alkyl or (C1-4 alkylene)-R^{11D};
   R^{11D} is hydroxy, C1-4 alkoxy, -COOH, C1-4 alkoxycarbonyl or -CONR^{7D}R^{8D};
   R^{5D} is a hydrogen atom or C 1-6 alkyl;
   R^{6D} is
   (i) C1-6 alkyl,
   (ii) C3-15 mono-, bi- or tri-carbocyclic ring or 3- to 15-membered mono-, bi- or tri-heterocyclic ring substituted by 1 to 5 R^{12D} groups or unsubstituted, or
   (iii) C1-6 alkyl, C2-6 alkenyl or C2-6 alkynyl substituted by C3-15 mono-, bi- or tri-carbocyclic ring or 3- to 15-membered mono-, bi- or tri-heterocyclic ring substituted by 1 to 5 R^{12D} groups or unsubstituted;
   R^{7D} and R^{8D} are each independently
   (i) a hydrogen atom,
   (ii) C1-6 alkyl,
   (iii) hydroxy,
   (iv) -COR^{17D},
   (v) C3-15 mono-, bi- or tri-carbocyclic ring or 3- to 15-membered mono-, bi- or tri-heterocyclic ring substituted by 1 to 5 R^{12D} groups or unsubstituted, or
   (vi) C1-4 alkyl substituted by C3-15 mono-, bi- or tri-carbocyclic ring or 3- to 15-membered mono-, bi- or tri-heterocyclic ring substituted by 1 to 5 R^{12D} groups or unsubstituted;
   R^{9D} is a hydrogen atom or C1-6 alkyl;
   R^{10D} is
   (i) a hydrogen atom,
   (ii) C1-6 alkyl,
   (iii) C3-15 mono-, bi- or tri-carbocyclic ring or 3-15 membered mono-, bi- or tri-heterocyclic ring substituted by 1 to 5 R^{12D} groups or unsubstituted, or
   (iv) C1-6 alkyl, C2-6 alkenyl or C2-6 alkynyl substituted with C3-15 mono-, bi- or tri-carbocyclic ring or 3- to 15-membered mono-, bi- or tri-heterocyclic ring substituted by 1 to 5 R^{12D} groups or unsubstituted;
   R^{12D} is (a) C1-6 alkyl, (b) C1-6 alkoxy, (c) C1-6 alkylthio, (d) a halogen atom, (e) CF₃, (f) cyano, (g) nitro, (h) hydroxy, (i) -COOR^{13D}, (j) -NHCOR^{13D}, (k) -SO₂R^{14D}, (l) - NR^{15D}R^{16D}, (m) C3-7 mono-carbocyclic ring substituted by C1-4 alkyl or oxo or unsubstituted, (n) 3- to 7-membered mono-heterocyclic ring substituted by C1-4 alkyl or oxo or unsubstituted or (o) C1-4 alkyl substituted by hydroxy, -COOR^{13D}, -NHCOR^{13D}, - SO₂R^{14D}, or -NR^{15D}R^{16D};
   R^{13D} is a hydrogen atom, C1-4 alkyl, phenyl, or phenyl(C1-4)alkyl;
   R^{14D} is C1-4 alkyl;
   R^{15D} and R^{16D} are each independently a hydrogen atom, C1-4 alkyl, phenyl, phenyl(C 1-4)alkyl;
   R^{17D} is C1-4 alkyl or phenyl;
   A^{D} is
   (i) a single bond,
   (ii) C1-6 alkylene,
   (iii) C2-6 alkenylene,
   (iv) C2-6 alkynylene,
   (v) -O-(C1-3 alkylene),
   (vi) -S-(C1-3 alkylene),
   (vii) -NR^{20D}-(C1-3 alkylene),
   (viii) -CONR^{21D}-(C1-3 alkylene),
   (ix) -(C1-3 alkylene)-O-(C1-3 alkylene),
   (x) -(C1-3 alkylene)-S-(C1-3 alkylene),
   (xi) -(C1-3 alkylene)-NR^{20D}-(C1-3 alkylene),
   (xii) -(C1-3 alkylene)-CONR^{21D}-(C1-3 alkylene),
   (xiii) -Cyc1^{D},
   (xiv) -(C1-4 alkylene)-Cyc1^{D}, or
   (xv) -Cyc1^{D}-(C1-4 alkylene),
   wherein the alkylene, alkenylene and alkynylene in A^{D} may be substituted by 1 to 6 substituents selected from the following substituents of (a)-(i):
   (a) C1-6 alkyl, (b) C1-6 alkoxy, (c) halogen atom, (d) CHF₂, (e) CF₃, (f) OCHF₂, (g) OCF₃, (h) hydroxy, (i) hydroxy(C1-4) alkyl;
   R^{20D} is a hydrogen atom, C1-4 alkyl, -SO₂(C1-4)alkyl or C2-5 acyl;
   R^{21D} is a hydrogen atom or C1-4 alkyl;
   Cyc1^{D} is C3-7 mono-carbocyclic ring or 3- to 7-membered mono-heterocyclic ring substituted with 1 to 4 substituents selected from C1-6 alkyl, C1-6 alkoxy, C1-6 alkylthio, C2-6 alkenyl, C2-6 alkynyl, halogen atom, CHF₂, CF₃, nitro and cyano or unsubstituted;
   B^{D} ring is C3-12 mono- or bi-carbocyclic ring or 3- to 12-membered mono- or bi-heterocyclic ring;
   R^{2D} is C1-6 alkyl, C1-6 alkoxy, C1-6 alkylthio, C2-6 alkenyl, C2-6 alkynyl, halogen atom, CHF₂, CF₃, nitro, cyano, phenyl or oxo;
   m^{D} is 0, 1 or 2,
   wherein
   when -D-R^{3D} binds to B^{D} ring at the ortho position based on -A^{D}-R^{1D}, then n^{D} is 1 or 2, and
   when -D-R^{3D} binds to B^{D} ring at the non-ortho position based on -A^{D}-R^{1D}, then n^{D} is 0, 1 or 2;
   Q^{D} is
   (1)
      (i) -(C1-4 alkylene, C2-4 alkenylene or C2-4 alkynylene)-Cyc2^{D},
      (ii) -(C1-4 alkylene)-Z^{D}-Cyc3^{D},
      (iii) C1-4 alkyl substituted by substituent(s) selected from -NR^{24D}R^{25D}, -S(O)_{pD}R^{26D}, cyano, -NR^{23D}COR^{27D}, -NR^{23D}SO₂R^{28D} and -NR^{23D}CONR^{24D}R^{25D}
      (iv) a group selected from C1-4 alkoxy(C1-4)alkoxy, -NR^{23D}COR^{27D}, -COR^{28D}, -OSO₂R^{28D}, -NR^{23D}SO₂R^{28D} and -NR^{23D}CONR^{24D}R^{25D},
      (v) C3-7 mono-carbocyclic ring or 3- to 6-membered mono-heterocyclic ring substituted with 1 to 5 R^{30D}'s, wherein one of the R^{30D}'s binds to the ring at the non 1-position,
      (vi) C8-15 mono-, bi- or tri-carbocyclic ring or 7- to 15-membered mono-, bi- or tri-heterocyclic ring substituted by 1 to 5 R^{30D}'s or unsubstituted,
      (vii) -T^{D}-Cyc5^{D} or
      (viii) a group selected from -L^{D}-Cyc6-1^{D}, -L^{D}-(C3-6 cycloalkyl), -L^{D}-CH₂-(C3-6 cycloalkyl), -L^{D}-(C2-4 alkylene)-Cyc6^{D}-2 and -L^{D}-(C1-4 alkylene)_{qD}-Cyc6^{D}-3 wherein the C3-6 cycloalkyl is substituted by 1 to 5 R^{30D}'s or unsubstituted,
   (2) (i) phenoxy,
      (ii) benzyloxy,
      (iii) hydroxy(C1-4)alkyl,
      (iv) C1-4 alkoxy(C 1-4)alkyl or
      (v) -(C1-4 alkylene)-O-benzyl, or
   (3) (i) C2-6 alkenyl,
      (ii) C2-6 alkynyl,
      (iii) C1-6 alkyl substituted by 1 to 3 halogen atoms,
      (iv) cyano,
      (v) nitro,
      (vi) -NR^{33D}R^{34D},
      (vii) -CONR^{33D}R^{34D},
      (viii) -S(O)_{pD}-(C1-4)alkynyl,
      (ix) -S(O)_{pD}-CHF₂,
      (x) -S(O)_{pD}-NR^{33D}R^{34D},
      (xi) -O-(C3-6)alkynyl,
      (xii) -O-CHF₂, or
      (xiii) C3-7 cycloalkyl;
   R^{22D} is a hydrogen atom, C1-4 alkyl, -SO₂-(C1-4)alkyl or C2-5 acyl;
   R^{23D} is a hydrogen atom, C1-4 alkyl, phenyl or phenyl(C1-4)alkyl;
   R^{24D} and R^{25D} are each independently a hydrogen atom, C1-4 alkyl, Cyc4^{D} or (C1-4 alkylene)-Cyc4^{D};
   R^{26D} is C1-4 alkyl or Cyc4^{D};
   R^{27D} is a hydrogen atom, C1-4 alkyl, -OR^{29D} or Cyc4^{D};
   R^{28D} is C1-4 alkyl, Cyc4^{D} or -(C1-4 alkylene)-Cyc4^{D};
   R^{29D} is a hydrogen atom, C1-4 alkyl, Cyc4^{D} or (C1-4 alkylene)-Cyc4^{D};
   R^{30D} is C1-8 alkyl, C1-8 alkoxy, C1-8 alkylthio, a halogen atom, CF₃, OCF₃, SCF₃, CHF₂, OCHF₂, SCHF₂, hydroxy, cyano, nitro, -NR^{31D}R^{32D}, -CONR^{31D}R^{32D}, formyl, C2-5 acyl, hydroxy(C1-4)alkyl, C1-4 alkoxy(C1-4)alkyl, C1-4 alkylthio(C1-4)alkyl, -(C1-4 alkylene)-CONR^{31D}R^{32D}, -SO₂(C1-4)alkyl, -NR^{23D}CO-(C1-4)alkyl,
   -NR^{23D}SO₂-(C1-4)alkyl, benzoyl, oxo, C3-7 mono-carbocyclic ring, 3- to 7-membered mono-heterocyclic ring, -(C1-4 alkylene)-NR^{31D}R^{32D}, -M^{D}-(C3-7 mono-carbocyclic ring) or -M^{D}-(3- to 7-membered mono-heterocyclic ring),
   wherein the C3-7 mono-carbocyclic ring and 3- to 7-membered mono-heterocyclic ring in R^{30D} may be substituted with 1 to 5 substituents selected from the following (a)-(1):
   (a) C1-6 alkyl, (b) C2-6 alkenyl, (c) C2-6 alkynyl, (d) C1-6 alkoxy, (e) C1-6 alkylthio, (f) halogen atom, (g) CHF₂, (h) CF₃, (i) nitro, (j) cyano, (k) hydroxy, (l) amino;
   M^{D} is -O-, -S-, C1-4 alkylene, -O-(C1-4 alkylene)-, -S-(C1-4 alkylene)-, -(C1-4 alkylene)-O-, or -(C1-4 alkylene)-S-;
   R^{31D} and R^{32D} are each independently a hydrogen atom or C1-4 alkyl;
   Cyc2^{D} is C3-15 mono-, bi- or tri-carbocyclic ring or 3- to 15-membered mono-, bi- or tri-heterocyclic ring substituted by 1 to 5 R^{30D}'s or unsubstituted;
   Z^{D} is -O-, -S(O)_{pD}-, -NR^{22D}-, -NR^{23D}CO-, -NR^{23D}SO₂-, -NR^{22D}-(C1-4 alkylene)-, - S(O)_{pD}-(C1-4 alkylene)-, -O-(C2-4 alkylene)-, -NR^{23D}CO-(C1-4 alkylene) or -NR^{23D}SO₂₋(C1-4 alkylene);
   p^{D} is 0, 1 or 2;
   Cyc3^{D} is C3-15 mono-, bi- or tri-carbocyclic ring or 3- to 15-membered mono-, bi- or tri-heterocyclic ring substituted by 1 to 5 R^{30D}'s or unsubstituted;
   Cyc4^{D} is C3-12 mono- or bi-carbocyclic ring or 3- to 12-membered mono- or bi-heterocyclic ring substituted by 1 to 5 R^{30D}'s or unsubstituted;
   T^{D} is -O-, -NR^{22D}-, -O-(C1-4 alkylene)-, -S(O)_{pD}-(C1-4 alkylene)- or -NR^{22D}-(C1-4 alkylene);
   Cyc5^{D} is 3- to 15-membered mono-, bi- or tri-heterocyclic ring substituted by 1 to 5 R^{30D}'s or unsubstituted;
   q^{D} is 0 or 1;
   L^{D} is -O- or -NR^{23D}-;
   Cyc6-1^{D} is phenyl or benzyl substituted by one or more R^{30D}'s;
   Cyc6-2^{D} is C3-6 mono-carbocyclic ring substituted by 1 to 5 R^{30D}'s or unsubstituted;
   Cyc6-3^{D} is C7-15 mono-, bi- or tri-carbocyclic ring substituted by 1 to 5 R^{30D}'s or unsubstituted;
   R^{33D} and R^{34D} are each independently a hydrogen atom, C1-4 alkyl, phenyl or benzyl, or
   NR^{33D}R^{34D} representing 3- to 6-membered mono-heterocyclic ring which may contain one nitrogen atom and optional one hetero atom selected from nitrogen, oxygen and sulfur atom;
   D^{D} is
   (1) 1- or 2-membered linker comprising atom(s) selected from carbon, nitrogen, oxygen and sulfur atom, which may contain a double bond or a triple bond and may be substituted by 1 to 4 R^{40D}'s,
   (2) 3- to 6-membered linker comprising atoms selected from carbon, nitrogen, oxygen and sulfur, which may contain double bond(s) or triple bond(s) and may be substituted by 1 to 12 R^{40D}'s, wherein R^{40D} substituted on the atom bound to R^{3D}, and R^{42D} which is a substituent of R^{3D} may be taken together to form -(CH₂)_{yD}- wherein y^{D} is 1 to 4, or
   (3) 7- to 10-membered linker comprising atoms selected from carbon, nitrogen, oxygen and sulfur atom, which may contain double bonds or triple bonds and may be substituted by 1 to 20 R^{40D}'s, wherein R^{40D} substituted on the atom binding to R^{3D}, and R^{42D} which is a substituent of R^{3D} may be taken together to form -(CH₂)_{yD}-;
   R^{40D} is (a) C1-8 alkyl, (b) C2-8 alkenyl, (c) C2-8 alkynyl, (d) oxo, (e) halogen atom, (f) CF₃, (g) hydroxy, (h) C1-6 alkoxy, (i) C2-6 alkenyloxy, (j) C2-6 alkynyloxy, (k) OCF₃, (l) -S(O)_{pD}-(C1-6)alkyl, (m) -S(O)_{pD}-(C2-6)alkenyl, (n) -S(O)_{pD}-(C2-6)alkynyl, (o) C2-5 acyl, (p) Cyc9^{D}, (q) C1-4 alkoxy(C1-4)alkoxy, (r) C1-8 alkyl, C2-8 alkenyl or C2-8 alkynyl substituted by 1 or 2 substituents selected from halogen atom, CF₃, OCF₃, hydroxy, cyano, C1-4 alkoxy, -S(O)_{pD}-(C1-6)alkyl, Cyc9^{D} and C1-4 alkoxy(C1-4)alkoxy, or
   two R^{40D}'s may be taken together with the atom of a linker to which they bind to form C3-15 mono-, bi- or tri-carbocyclic ring or 3- to 15-membered mono-, bi- or tri-heterocyclic ring containing 1 to 2 hetero atoms selected from O, S, SO₂ and N, wherein the carbocyclic ring and the heterocyclic ring may be substituted by 1 to 3 substituents selected from C1-4 alkyl, C1-4 alkoxy, C2-5 acyl, SO₂(C1-4 alkyl), phenyl and phenyl(C1-4) alkyl;
   Cyc9^{D} is C3-6 mono-carbocyclic ring or 3- to 6-membered mono-heterocyclic ring substituted by 1 to 5 R^{41D}'s or unsubstituted;
   R^{41D} is C1-4 alkyl, C1-4 alkoxy, C1-4 alkylthio, C1-4 alkoxy(C1-4)alkyl, a halogen atom, CF₃, OCF₃, SCF₃, hydroxy, cyano, formyl, C2-5 acyl, -SO₂-(C1-4)alkyl, - NR^{23D}CO-(C1-4)alkyl, benzyl or oxo;
   R^{3D} is
   (1) C1-6 alkyl or
   (2) C3-15 mono-, bi- or tri-carbocyclic ring or 3- to 15-membered mono-, bi- or tri-heterocyclic ring substituted by 1 to 5 R^{42D}'s or unsubstituted;
   R^{42D} is (a) C1-6 alkyl, (b) C1-6 alkoxy, (c) C1-6 alkylthio, (d) a halogen atom, (e) cyano, (f) CF₃, (g) CHF₂, (h) OCF₃, (i) OCHF₂, (j) SCF₃, (k) -NR^{43D}R^{44D}, (1) -SO₂R^{45D}, (m) -NR^{46D}COR^{47D}, (n) hydroxy, (o) oxo, (p) C1-4 alkoxy(C1-4)alkyl, (q) Cyc10^{D}, (r) C1-6 alkylene-Cyc10^{D}, (s) -CO-Cyc10^{D}, (t) -W^{D}-Cyc10^{D}, (u) -(C1-6 alkylene)-W^{D}-Cyc10^{D}, (v) -W^{D}-(C1-6 alkylene)-Cyc10^{D} or (w) -(C1-6 alkylene)-W^{D}-(C1-6 alkylene)-Cyc10^{D};
   R^{43D} and R^{44D} are each independently a hydrogen atom or C1-4 alkyl;
   R^{45D} is C1-4 alkyl;
   R^{46D} is a hydrogen atom or C1-4 alkyl;
   R^{47D} is a hydrogen atom or C1-4 alkyl;
   Cyc10^{D} is C3-12 mono- or bi-carbocyclic ring or 3- to 12-membered mono- or bi-heterocyclic ring substituted by 1 to 5 substituents selected from the following (a)-(j) or unsubstituted:
   (a) C1-4 alkyl, (b) C2-5 acyl, (c) C1-4 alkoxy, (d) a halogen atom, (e) hydroxy, (f) nitro, (g) cyano, (h) amine, (i) CF₃, (j) OCF₃;
   W^{D} is -O-, -S(O)_{pD}- or -NR^{48D}-;
   R^{48D} is a hydrogen atom or C1-4 alkyl, a salt thereof, a solvate thereof or a prodrug thereof, and
   a compound represented by formula (E) 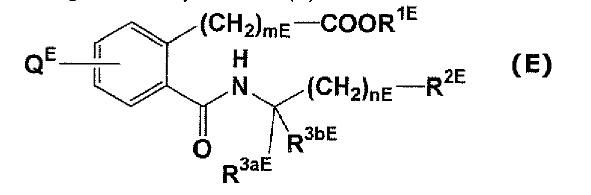 wherein R^{1E} is a hydrogen atom or C1-4 alkyl;
   R^{2E} is phenyl, naphthyl, benzofuranyl or benzothienyl substituted by 1 or 2 substituents selected from C1-4 alkyl or a halogen atom or unsubstituted;
   Q^{E} is (i) -CH₂-O-Cyc1^{E}, (ii) -CH₂-Cyc2^{E} or (iii) -L-Cyc3;
   Cyc1^{E} is phenyl or pyridyl substituted by one or two R^{4E}'s or unsubstituted;
   Cyc2^{E} is indolyl substituted by one or two R^{4E}'s or unsubstituted;
   Cyc3^{E} is phenyl substituted by one or two R^{4E}'s or unsubstituted;
   L is -O- or -NH-;
   R^{3a} and R^{3b} are each independently a hydrogen atom or C1-4 alkyl, or are taken together with the carbon atom to which R^{3aE} and R^{3bE} are attached to form tetrahydro-2H-pyran;
   mE is 2 or 3;
   nE is 0, 1 or 2;
   R^{4E} is C1-4 alkyl, C1-4 alkylthio, a halogen atom or cyano, or when Cyc3^{E} is phenyl substituted by two R^{4E}'s, and two R^{4E}'s, together with phenyl, may form 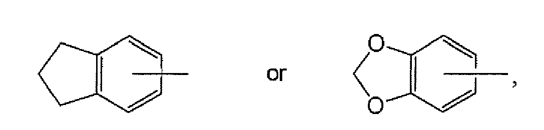 a salt thereof, a solvate thereof or a prodrug thereof.
14. The agent for preventing and/or treating urinary tract disease according to 13 above, wherein the compound is N-(3,4-difluorophenylsulfonyl)-3-(2-(2-(naphthalen-2-yl)ethoxy)-4-(3-cyanophenoxymethyl)phenyl)propanamide, 3-[4-[(2,5-dimethylphenoxy)methyl]-2-({[(1R)-1-(3,5-diethylphenyl)-3-methylbutyl]amino}carbonyl)phenyl]propanoic acid, 3-(2-(((1R)-3-methyl-1-(3,5-dimethylphenyl)butyl)carbamoyl)-4-(2,5-difluorophenoxymethyl)phenyl)propanoic acid, or 3-(2-((((1R)-1-(3,5-dimethylphenyl)-3-methylbutyl)amino)carbonyl)-4-(5-fluoro-2-methylphenoxymethyl)phenyl)propanoic acid.
15. The agent for preventing and/or treating urinary tract disease according to 1 above, wherein the EP₁ antagonist and the EP₃ antagonist are used at low doses.
16. An agent for preventing and/or treating urinary tract disease, which comprises a compound having antagonism to EP₁ and antagonism to EP₃.
17. A method for preventing and/or treating urinary tract disease, which comprises administering an effective amount of a medicament comprising a combination of EP₁ antagonist and EP₃ antagonist to a maminal.
18. A method for preventing and/or treating urinary tract disease, which comprises administering an effective amount of a compound having antagonism to EP₁ and antagonism to EP₃ to a mammal.
19. Use of a combination of EP₁ antagonist and EP₃ antagonist, for the preparation of an agent for preventing and/or treating urinary tract disease.
20. Use of a compound having antagonism to EP₁ and antagonism to EP₃, for the preparation of an agent for preventing and/or treating urinary tract disease.

EP₁ antagonist or EP₃ antagonist used in the present invention includes any compound which has antagonism to EP₁ or antagonism to EP₃. In addition, not only EP₁ antagonist or EP₃ antagonist that has known but also the one that will be newly found in the future are included.

As EP₁ antagonist used in the present invention, for example, (1) compounds described in WO98/27053, (2) compounds described in EP878465, (3) compounds described in WO02/72564, (4) compounds described in WO97/00863, (5) compounds described in WO97/00864, (6) compounds described in EP480641, (7) compounds described in EP534667, (8) compounds described in WO96/03380, (9) compounds described in WO96/06822, (10) compounds described in WO96/11902, (11) compounds described in EP752421, (12) compounds described in US5504077, (13) compounds described in EP694546, (14) compounds described in US5441950, (15) compounds described in US5420270, (16) compounds described in US5354747, (17) compounds described in US5354746, (18) compounds described in US5324722, (19) compounds described in US5304644, (20) compounds described in compounds described in US5281590, (21) compounds described in WO93/13082, (22) compounds described in EP539977, (23) compounds described in WO93/07132 (24) compounds described in EP512400, (25) compounds described in EP512399, (26) compounds described in EP218077, (27) compounds described in EP193822, (28) compounds described in WO92/19617, (29) compounds described in US4132847, (30) compounds described in EP300676, (31) compounds described in US4775680, (32) compounds described in EP160408, (33) EP₁ antagonists in compounds described in WO99/47479, (34) EP₁ antagonists in compounds described in WO00/20371, (35) EP₁ antagonists in compounds described in WO01/19814, (36) EP₁ antagonists in compounds described in WO01/19819, (37) EP₁ antagonists in compounds described in WO03/33470, (38) EP₁ antagonists in compounds described in WO03/101959, and (39) EP₁ antagonists in compounds described in WO04/039753 are used.

Concretely, example compounds, salts thereof, solvates thereof or prodrugs thereof described in each specification of above (1)-(39) are used as EP₁ antagonists used in the present invention.

In the present invention, preferably EP₁ antagonists are compounds described below.
(1) compounds represented by formula (A) 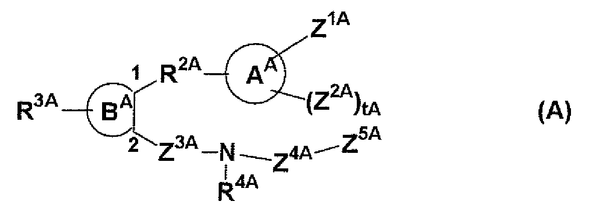 wherein 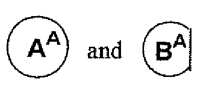 are each independently C5-15 carbocyclic ring or 5-to 7-membered heterocyclic ring having 1 or 2 oxygen, sulfur or nitrogen atoms;
   Z^{1A} is a group represented by -COR^{1A}, -C1-4 alkylene-COR^{1A}, -CH=CH-COR^{1A}, -C≡C-COR^{1A}, -O-C1-3 alkylene-COR^{1A} wherein R^{1A} is hydroxy, C1-4 alkoxy or a group represented by formula NR^{6A}R^{7A} wherein R^{6A} and R^{7A} are independently hydrogen atom or C1-4 alkyl or -C1-5 alkylene-OH; Z^{2A} is a hydrogen atom, C1-4 alkyl, C1-4 alkoxy, nitro, halogen, trifluoromethyl, trifluoromethoxy, hydorxy or a group represented by formula COR^{1A} wherein R^{1A} has the same meaning as described above; Z^{3A} is a single bond or C1-4 alkylene; Z^{4A} is SO₂ or CO; Z^{5A} is (1) C1-8 alkyl, C2-8 alkenyl, C2-8 alkynyl, (2) phenyl, C3-7 cycloalkyl, 5- to 7-membered heterocyclic ring having 1 or 2 oxygen, sulfur or nitrogen atoms, (3) C1-4 alkyl, C2-4 alkenyl or C2-4 alkynyl substituted by phenyl or C3-7 cycloalkyl wherein phenyl, C3-7 cycloalkyl and 5- to 7-membered heterocyclic ring having 1 or 2 oxygen, sulfur or nitrogen atoms in above-described (2) and (3) may by substituted by 1 to 5 R^{5A} groups wherein multiple R^{5A}'s are independently a hydrogen atom, C1-6 alkyl, C1-6 alkoxy, C1-6 alkylthio, nitro, halogen, trifluoromethyl, trifluoromethoxy or hydroxy; R^{2A} is CONR^{8A} NR^{8A}CO, CONR^{8A}-C1-4 alkylene, C1-4 alkylene-CONR^{8A}, NR^{8A}CO-C1-4 alkylene, C1-4 alkylene-NR^{8A}CO, C1-3 alkylene-CONR^{8A}-C1-3 alkylene, C1-3 alkylene-NR^{8A}CO-C1-3 alkylene wherein R^{8A} is hydrogen atom or C1-4 alkyl, O, S, NZ^{6A} wherein Z^{6A} is hydrogen atom or C1-4 alkyl, Z^{7A}-C1-4 alkylene, C1-4 alkylene-Z^{7A}, C1-3 alkylene-Z^{7A}-C1-3 alkylene wherein Z^{7A} is O, S or NZ^{6A} wherein Z^{6A} has the same meaning as described above, CO, CO-C1-4 alkylene, C1-4 alkylene-CO, C1-3 alkylene-CO-C1-3 alkylene, C2-4 alkylene, C2-4 alkenylene or C2-4 alkynylene;
   R^{3A} is a hydrogen atom, C1-6 alkyl, C1-6 alkoxy, C1-6 alkylthio, nitoro, halogen, trifluromethyl, trifluoromethoxy, hydroxy or hydroxymethyl; R^{4A} is (1) a hydrogen atom, (2) C1-8 alkyl, C2-8 alkenyl, C2-8 alkynyl, (3) C1-6 alkyl substituted by 1 or 2 group(s) selected from COOZ^{8A}, CONZ^{9A}Z^{10A}, OZ^{8A} wherein Z^{8A}, Z^{9A} and Z^{10A} are independently a hydrogen atom or C1-4 alkyl, C1-4 alkoxy-C1-4 alkoxy, (4) C3-7 cycloalkyl, (5) C1-4 alkyl, C2-4 alkenyl or C2-4 alkynyl substituted by phenyl or C3-7 cycloalkyl wherein phenyl or C3-7 cycloalkyl in above-described (4) and (5) may be substituted by 1 to 5 R^{5A} groups wherein R^{5A} has the same meaning as described above; n^{A} and t^{A} are each independently an integer from 1 to 4, and wherein (1) R^{2A} and Z^{3A} are each connected at the 1- or 2-position of 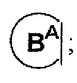 and (2) when 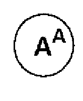 is benzene and (Z^{2A})_{tA} is other than COR^{1A}, Z^{1A} is connected at the 3- or 4-position of benzene, salts thereof, solvates thereof or prodrugs thereof described in WO98/27053,
(2) compounds represented by formula (B) 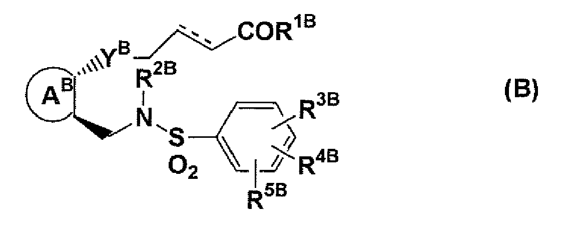 wherein 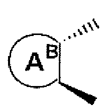 is a group represented by formula 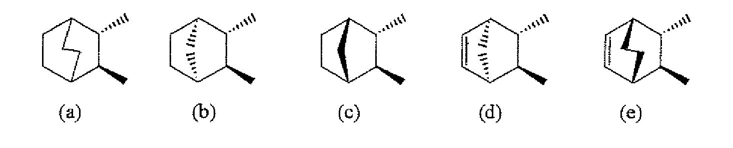 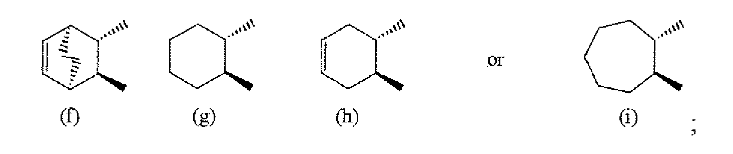 R^{1B} is hydroxy, C1-4 alkoxy or a group represented by formula NR^{6B}R^{7B} wherein R^{6B} and R^{7B} are each independently a hydrogen atom or C1-4 alkyl; R^{2B} is a hydrogen atom or C1-4 alkyl, R^{3B} and R^{4B} are C1-4 alkyl, halogen atom or trifluoromethyl; R^{5B} is a hydrogen atom, C1-4 alkyl, a halogen atom or trifluoromethyl; Y^{B} is cis-vinylene or trans-vinylene, symbol 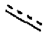 is a single bond or double bond, with the proviso that, when 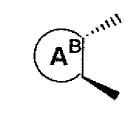 is formula 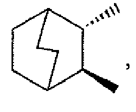 R^{1B} is hydroxy or C1-4 alkoxy, R^{2B} is a hydrogen atom, Y^{B} is cis-vinylene and symbol 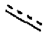 is a single bond; 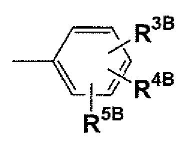 is not 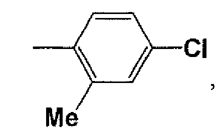 salts thereof, solvates thereof or prodrugs thereof described in EP878465, and
(3) compounds represented by formula (C) 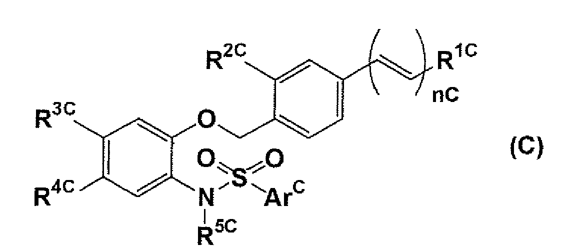
wherein, R^{1C} is COOH, 5-tetrazolyl, 5-oxo-1,2,4-oxadiazolyl, CH₂OH or 5-oxo-1,2,4-thiadiazolyl; R^{2C} is hydrogen, methyl, methoxy or chloro, R^{3C} and R^{4C} are each a combination of (1) methyl and methyl, (2) methyl and chloro, (3) chloro and methyl or (4) trifluoromethyl and hydrogen, or are taken together with the carbon atom to which they are attached to form (5) cyclopentene, (6) cyclohexene or (7) benzene; R^{5C} is isopropyl, isobutyl, 2-methyl-2-propenyl, cyclopropylmethyl, methyl, ethyl, propyl, 2-propenyl or 2-hydroxy-2-methylpropyl; Ar^{C} is thiazolyl which may be substituted by methyl, pyridyl or 5-methyl-2-furyl; n^{C} is 0 or 1, with the proviso that when R^{1C} is 5-tetrazolyl, 5-oxo-1,2,4-oxadiazolyl or 5-oxo-1,2,4-thiadiazolyl, n^{C} is 0, alkyl esters thereof, salts thereof or prodrugs thereof described in WO02/72564.

Concretely, example compounds or salts thereof described in specifications of above (1)-(3). More preferably,
(1) 4-[2-(N-isobutyl-2-furanylsulfonylamino)-5-trifluoromethylphenoxymethyl]cinnamic acid,
(2) 6-[(2S,3S)-3-(4-chloro-2-methylphenylsulfonylaminomethyl)bicyclo[2.2.2]octan-2-yl]-5Z-hexenoic acid,
(3) 3-methyl-4-[2-[N-isobutyl-N-(5-methyl-2-furylsulfonyl)amino]-4,5-dimethylphenoxymethyl]benzoic acid,
(4) 4-[2-[N-isopropyl-N-(5-methyl-2-furylsulfonyl)amino]-5-trifluoromethylphenoxymethyl]benzoic acid,
(5) 4-[2-[N-isobutyl-N-(5-methyl-2-furylsulfonyl)amino]-4,5-dimethylphenoxymethyl]benzoic acid,
(6) 3-chloro-4-[2-[N-isobutyl-N-(5-methyl-2-furylsulfonyl)amino]-4,5-dimethylphenoxymethyl]benzoic acid,
(7) 4-[6-[N-isobutyl-N-(5-methyl-2-furylsulfonyl)amino]indan-5-yloxymethyl]benzoic acid,
(8) 4-[6-[N-isobutyl-N-(5-methyl-2-furylsulfonyl)amino]indan-5-yloxymethyl]cinnamic acid,
(9) 3-methyl-4-[6-[N-isobutyl-N-(5-methyl-2-furylsulfonyl)amino]indan-5-yloxymethyl]benzoic acid,
(10) 4-[2-[N-isobutyl-N-(2-thiazolylsulfonyl)amino]-5-trifluoromethylphenoxymethyl]cinnamic acid,
(11) 4-[2-[N-isobutyl-N-(4-methyl-2-thiazolylsulfonyl)amino]-5-trifluoromethylphenoxymethyl]cinnamic acid,
(12) 3-methyl-4-[2-[N-isobutyl-N-(4-methyl-2-thiazolylsulfonyl)amino]-4,5-dimethylphenoxymethyl]benzoic acid,
(13) 3-methyl-4-[2-[N-isobutyl-N-(4-methyl-2-thiazolylsulfonyl)amino]-4,5-dimethylphenoxymethyl]cinnamic acid,
(14) 4-[2-[N-isobutyl-N-(4-methyl-2-thiazolylsulfonyl)amino]-4,5-dimethylphenoxymethyl]cinnamic acid,
(15) 3-chloro-4-[2-[N-isobutyl-N-(4-methyl-2-thiazolylsulfonyl)amino]-4,5-dimethylphenoxymethyl]cinnamic acid,
(16) 4-[6-[N-isobutyl-N-(4-methyl-2-thiazolylsulfonyl)amino]indan-5-yloxymethyl]benzoic acid,
(17) 3-methyl-4-[6-[N-isobutyl-N-(4-methyl-2-thiazolylsulfonyl)amino]indan-5-yloxymethyl]benzoic acid
(18) 3-methyl-4-[6-[N-isobutyl-N-(4-methyl-2-thiazolylsulfonyl)amino]indan-5-yloxymethyl]cinnamic acid,
(19) 4-[6-[N-isopropyl-N-(5-methyl-2-furylsulfonyl)amino]indan-5-yloxymethyl]cinnamic acid,
(20) 4-[6-[N-isobutyl-N-(2-thiazolylsulfonyl)amino]indan-5-yloxymethyl]benzoic acid,
(21) 4-[6-[N-isopropyl-N-(4-methyl-2-thiazolylsulfonyl)amino]indan-5-yloxymethyl]cinnamic acid,
(22) 3-methyl-4-[6-[N-isopropyl-N-(4-methyl-2-thiazolylsulfonyl)amino]indan-5-yloxymethyl]cinnamic acid,
(23) 4-[2-[N-isobutyl-N-(2-thiazolylsulfonyl)amino]-4,5-dimethylphenoxymethyl]benzoic acid,
(24) 3-methyl-4-[2-[N-isobutyl-N-(2-thiazolylsulfonyl)amino]-4,5-dimethylphenoxymethyl]benzoic acid,
(25) 4-[4,5-dimethyl-2-[N-(5-methyl-2-furylsulfonyl)-N-(2-methyl-2-propenyl)amino]phenoxymethyl]-3-methylbenzoic acid,
(26) 3-methyl-4-[6-[N-(5-methyl-2-furylsulfonyl)-N-propylamino]indan-5-yloxymethyl]benzoic acid,
(27) 3-methyl-4-[6-[N-(5-methyl-2-furylsulfonyl)-N-(2-propenyl)amino]indan-5-yloxymethyl]benzoic acid,
(28) 4-[4,5-dimethyl-2-[N-(5-methyl-2-furylsulfonyl)-N-propylamino]phenoxymethyl]benzoic acid,
(29) 4-[4,5-dimethyl-2-[N-(5-methyl-2-furylsulfonyl)-N-(2-propenyl)amino]phenoxymethyl]-3-methylbenzoic acid,
(30) 4-[2-[N-cyclopropylmethyl-N-(5-methyl-2-furylsulfonyl)amino]-4,5-dimethylphenoxymethyl]benzoic acid,
(31) 4-[6-[N-(2-hydroxy-2-methylpropyl)-N-(5-methyl-2-furylsulfonyl)amino]indan-5-yloxymethyl]cinnamic acid,
(32) 3-methyl-4-[6-[N-(4-methyl-2-thiazolylsulfonyl)-N-propylamino]indan-5-yloxymethyl]cinnamic acid, salts thereof, specifically sodium salts thereof, solvates thereof or prodrugs thereof are used.

As EP₃ antagonist used in the present invention, for example, (40) EP₃ antagonist in compounds described in WO01/62708, (41) EP₃ antagonist in compounds described in WO02/16311, (42) EP₃ antagonist in compounds described in WO02/20462, (43) EP₃ antagonist in compounds described in WO03/16254, (44) EP₃ antagonist in compounds described in WO99/47479, (45) EP₃ antagonist in compounds described in WO00/20371, (46) EP₃ antagonist in compounds described in WO01/19814, and (47) EP₃ antagonist in compounds described in WO01/19819 are used.

Concretely, example compounds or salts thereof described in each specification of above (40)-(47) are used as EP₃ antagonists used in the present invention.

Preferably EP₃ antagonists are compounds represented by formula (D) 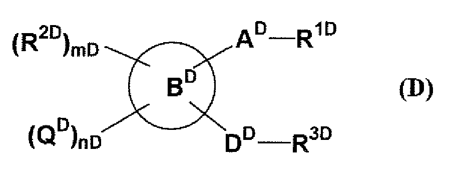 wherein R^{1D} is -COOH, -COOR^{4D}, -CH₂OH, -CONR^{5D}SO₂R^{6D}, -CONR^{7D}R^{8D}, -CH₂NR^{5D}SO₂R^{6D}, -CH₂NR^{9D}COR^{10D}, -CH₂NR^{9D}CONR^{5D}SO₂R^{6D}, -CH₂SO₂NR^{9D}COR^{10D}, -CH₂OCONR^{5D}SO₂R^{6D}, tetrazole, 1,2,4-oxadiazol-5-one, 1,2,4-oxadiazole-5-thione, 1,2,4-thiadiazol-5-one, 1,3-thiazolidine-2,4-dione, or 1,2,3,5-oxathiadiazol-2-one;
R^{4D} is C1-6 alkyl or (C1-4 alkylene)-R^{11D};
R^{11D} is hydroxy, C1-4 alkoxy, -COOH, C1-4 alkoxycarbonyl or -CONR^{7D}R^{8D};
R^{5D} is a hydrogen atom or C1-6 alkyl;
R^{6D} is
(i) C1-6 alkyl;
(ii) C3-15 mono-, bi- or tri-carbocyclic ring or 3- to 15-membered mono-, bi- or tri-heterocyclic ring substituted by 1 to 5 R^{12D} groups or unsubstituted, or
(iii) C1-6 alkyl, C2-6 alkenyl or C2-6 alkynyl substituted by C3-15 mono-, bi- or tri-carbocyclic ring or 3- to 15-membered mono-, bi- or tri-heterocyclic ring substituted by 1 to 5 R^{12D} groups or unsubstituted;
R^{7D} and R^{8D} are each independently
(i) a hydrogen atom,
(ii) C1-6 alkyl,
(iii) hydroxy,
(iv) -COR^{17D},
(v) C3-15 mono-, bi- or tri-carbocyclic ring or 3- to 15-membered mono-, bi- or tri-heterocyclic ring substituted by 1-5 R^{12D} or unsubstituted, or
(vi) C1-4 alkyl substituted by C3-15 mono-, bi- or tri-carbocyclic ring or 3- to 15-membered mono-, bi- or tri-heterocyclic ring substituted by 1 to 5 R^{12D} groups or unsubstituted;
R^{9D} is a hydrogen atom or C1-6 alkyl;
R^{10D} is
(i) a hydrogen atom,
(ii) C1-6 alkyl,
(iii) C3-15 mono-, bi- or tri-carbocyclic ring or 3- to 15-membered mono-, bi- or tri-heterocyclic ring substituted by 1 to 5 R^{12D} groups or unsubstituted, or
(iv) C1-6 alkyl, C2-6 alkenyl or C2-6 alkynyl substituted with C3-15 mono-, bi- or tri-carbocyclic ring or 3- to 15-membered mono-, bi- or tri-heterocyclic ring substituted by 1 to 5 R^{12D} groups or unsubstituted;
R^{12D} is (a) C1-6 alkyl, (b) C1-6 alkoxy, (c) C1-6 alkylthio, (d) a halogen atom, (e) CF₃, (f) cyano, (g) nitro, (h) hydroxy, (i) -COOR^{13D}, (j) -NHCOR^{13D}, (k) -SO₂R^{14D}, (l) -NR^{15D}R^{16D}, (m) C3-7 mono-carbocyclic ring substituted by C1-4 alkyl or oxo or unsubstituted, (n) 3- to 7-membered mono-heterocyclic ring substituted by C1-4 alkyl or oxo or unsubstituted or (o) C1-4 alkyl substituted by hydroxy, -COOR^{13D}, -NHCOR^{13D}, - SO₂R^{14D}, or -NR^{15D}R^{16D};
R^{13D} is a hydrogen atom, C1-4 alkyl phenyl, phenyl(C1-4)alkyl; R^{14D} is C1-4 alkyl;
R^{15D} and R^{16D} are each independently a hydrogen atom, C1-4 alkyl, phenyl, phenyl(C1-4)alkyl;
R^{17D}is C1-4 alkyl or phenyl;
A^{D} is
(i) a single bond,
(ii) C1-alkylene,
(iii) C2-6 alkenylene,
(iv) C2-6 alkynylene,
(v) -O-(C1-3 alkylene),
(vi) -S-(C1-3 alkylene),
(vii) -NR^{20D}-(C1-3 alkylene),
(viii) -CONR^{21D}-(C1-3 alkylene),
(ix) -(C1-3 alkylene)-O-(C1-3 alkylene),
(x) -(C1-3 alkylene)-S-(C1-3 alkylene),
(xi) -(C1-3 alkylene)-NR^{20D}-(C1-3 alkylene),
(xii) -(C1-3 alkylene)-CONR^{21D}-(C1-3 alkylene),
(xiii) q-Cyc1^{D},
(xiv) q-(C1-4 alkylene)-Cyc1^{D}, or
(xv) q-Cyc1^{D}-(C1-4 alkylene),
wherein the alkylene, alkenylene and alkynylene in A^{D} may be substituted by 1 to 6 substituents selected from the following substituents of (a)-(i):
(a) C1-6 alkyl, (b) C1-6 alkoxy, (c) halogen atom, (d) CHF₂, (e) CF₃, (f) OCHF₂, (g) OCF₃, (h) hydroxy, (i) hydroxy(C1-4) alkyl;
R^{20D} is a hydrogen atom, C1-4 alkyl, -SO₂(C1-4)alkyl or C2-5 acyl;
R^{21D} is a hydrogen atom or C1-4 alkyl;
Cyc1^{D} is C3-7 mono-carbocyclic ring or 3- to 7-membered mono-heterocyclic ring substituted with 1 to 4 substituents selected from C1-6 alkyl, C1-6 alkoxy, C1-6 alkylthio, C2-6 alkenyl, C2-6 alkynyl, halogen atom, CHF₂, CF₃, nitro and cyano or unsubstituted;
B^{D} ring is C3-12 mono- or bi-carbocyclic ring or 3- to 12-membered mono- or bi-heterocyclic ring;
R^{2D} is C1-6 alkyl, C1-6 alkoxy, C1-6 alkylthio, C2-6 alkenyl, C2-6 alkynyl, halogen atom, CHF₂, CF₃, nitro, cyano, phenyl or oxo;
m^{D} is 0, 1 or 2,
wherein, when -D-R^{3D} binds to B^{D} ring at the ortho position based on -A^{D}-R^{1D}, then n^{D} is 1 or 2, and
when -D-R^{3D} binds to B^{D} ring at the non-ortho position based on -A^{D}-R^{1D}, then n^{D} is 0, 1 or 2;
Q^{D} is
(1) (i) -(C1-4 alkylene, C2-4 alkenylene or C2-4 alkynylene)-Cyc2^{D},
   (ii) -(C1-4 alkylene)-Z^{D}-Cyc3^{D},
   (iii) C1-4 alkyl substituted by substituent(s) selected from -NR^{24D}R^{25D}, -S(O)_{pD}R^{26D}, cyano, -NR^{23D}COR^{27D}, -NR^{23D}SO₂R^{28D} and -NR^{23D}CONR^{24D}R^{25D}
   (iv) a group selected from C1-4 alkoxy(C1-4)alkoxy, -NR^{23D}COR^{27D}, -COR^{28D}, -OSO₂R^{28D}, -NR^{23D}SO₂R^{28D} and -NR^{23D}CONR^{24D}R^{25D},
   (v) C3-7 mono-carbocyclic ring or 3- to 6-membered mono-heterocyclic ring substituted with 1 to 5 R^{30D}'s, wherein one of the R^{30D}'s binds to the ring at the non 1-position,
   (vi) C8-15 mono-, bi- or tri-carbocyclic ring or 7- to 15-membered mono-, bi- or tri-heterocyclic ring substituted by 1 to 5 R^{30D}'s or unsubstituted,
   (vii) -T^{D}-Cyc5^{D} or
   (viii) a group selected from -L^{D}-Cyc6-1^{D}, -L^{D}-(C3-6 cycloalkyl), -L^{D}-CH₂-(C3-6 cycloalkyl), -L^{D}-(C2-4 alkylene)-Cyc6^{D}-2 and -L^{D}-(C1-4 alkylene)_{qD}-Cyc6^{D}-3 wherein the C3-6 cycloalkyl is substituted by 1 to 5 R^{30D}'s or unsubstituted,
(2) (i) phenoxy,
   (ii) benzyloxy,
   (iii) hydroxy(C1-4)alkyl,
   (iv) C1-4 alkoxy(C1-4)alkyl or
   (v) -(C1-4 alkylene)-O-benzyl, or
   (3) (i) C2-6 alkenyl,
   (ii) C2-6 alkynyl,
   (iii) C1-6 alkyl substituted by 1 to 3 halogen atoms,
   (iv) cyano,
   (v) nitro,
   (vi) -NR^{33D}R^{34D},
   (vii) -CONR^{33D}R^{34D},
   (viii) -S(O)_{pD}-(C1-4)alkynyl,
   (ix) -S(O)_{pD}-CHF₂,
   (x) -S(O)_{pD}-NR^{33D}R^{34D},
   (xi) -O-(C3-6)alkynyl,
   (xii) -O-CHF₂, or
   (xiii) C3-7 cycloalkyl;
   R^{22D} is a hydrogen atom, C1-4 alkyl, -SO₂-(C1-4)alkyl or C2-5 acyl;
   R^{23D} is a hydrogen atom, C1-4 alkyl, phenyl or phenyl(C1-4)alkyl;
   R^{24D} and R^{25D} are each independently a hydrogen atom, C1-4 alkyl, Cyc4^{D} or (C1-4 alkylene)-Cyc4^{D};
   R^{26D} is C 1-4 alkyl or Cyc4^{D};
   R^{27D} is a hydrogen atom, C1-4 alkyl, -OR^{29D} or Cyc4^{D};
   R^{28D} is C1-4 alkyl, Cyc4^{D} or-(C1-4 alkylene)-Cyc4^{D};
   R^{29D} is a hydrogen atom, C1-4 alkyl, Cyc4^{D} or (C1-4 alkylene)-Cyc4^{D};
   R^{30D} is C1-8 alkyl, C1-8 alkoxy, C1-8 alkylthio, a halogen atom, CF₃, OCF₃, SCF₃, CHF₂, OCHF₂, SCHF₂, hydroxy, cyano, nitro, -NR^{31D}R^{32D}, -CONR^{31D}R^{32D}, formyl, C2-5 acyl, hydroxy(C1-4)alkyl, C1-4 alkoxy(C1-4)alkyl, C1-4 alkylthio(C1-4)alkyl, -(C1-4 alkylene)-CONR^{31D}R^{32D}, -SO₂(C1-4)alkyl, -NR^{23D}CO-(C1-4)alkyl, -NR^{23D}SO₂-(C1-4)alkyl, benzoyl, oxo, C3-7 mono-carbocyclic ring, 3- to 7-membered mono-heterocyclic ring, -(C1-4 alkylene)-NR^{31D}R^{32D}, -M^{D}-(C3-7 mono-carbocyclic ring) or -M^{D}-(3- to 7-membered mono-heterocyclic ring),
wherein the C3-7 mono-carbocyclic ring and 3- to 7-membered mono-heterocyclic ring in R^{30D} may be substituted with 1 to 5 substituents selected from the following (a)-(l):
(a) C1-6 alkyl, (b) C2-6 alkenyl, (c) C2-6 alkynyl, (d) C1-6 alkoxy, (e) C1-6 alkylthio, (f) a halogen atom, (g) CHF₂, (h) CF₃, (i) nitro, (j) cyano, (k) hydroxy, (l) amino;
M^{D} is -O-, -S-, C1-4 alkylene, -O-(C1-4 alkylene)-, -S-(C1-4 alkylene)-, -(C1-4 alkylene)-O-, or -(C1-4 alkylene)-S-;
R^{31D} and R^{32D} are each independently a hydrogen atom or C1-4 alkyl;
Cyc2^{D} is C3-15 mono-, bi- or tri-carbocyclic ring or 3- to 15-membered mono-, bi- or tri-heterocyclic ring substituted by 1 to 5 R^{30D}'s or unsubstituted,
Z^{D} is -O-, -S(O)_{pD}-, -NR^{22D}-, -NR^{23D}CO-, -NR^{23D}SO₂-, -N^{R22D}-(C1-4 alkylene)-, -S(O)_{pD}-(C1-4 alkylene)-, -O-(C2-4 alkylene)-, -NR^{23D}CO-(C1-4 alkylene) or -NR^{23D}SO₂₋(C1-4 alkylene);
p^{D} is 0, 1 or 2;
Cyc3^{D} is C3-15 mono-, bi- or tri-carbocyclic ring or 3- to 15-membered mono-, bi- or tri-heterocyclic ring substituted by 1 to 5 R^{30D}'s or unsubstituted;
Cyc4^{D} is C3-12 mono- or bi-carbocyclic ring or 3- to 12-membered mono- or bi-heterocyclic ring substituted by 1 to 5 R^{30D}'s or unsubstituted;
T^{D} is -O-, -NR^{22D}-, -O-(C1-4 alkylene)-, -S(O)_{pD}-(C1-4 alkylene)- or -NR^{22D}-(C1-4 alkylene);
Cyc5^{D} is 3- to 15-membered mono-, bi- or tri-heterocyclic ring substituted by 1 to 5 R^{30D}'s or unsubstituted;
q^{D} is 0 or 1;
L^{D} is -O- or -NR^{23D}-;
Cyc6-1^{D} is phenyl or benzyl substituted by one or more R^{30D}'s;
Cyc6-2^{D} is C3-6 mono-carbocyclic ring substituted by 1 to 5 R^{30D}'s or unsubstituted;
Cyc6-3^{D} is C7-15 mono-, bi- or tri-carbocyclic ring substituted by 1 to 5 R^{30D}'s or unsubstituted;
R^{33D} and R^{34D} are each independently a hydrogen atom, C1-4 alkyl, phenyl or benzyl, or
NR^{33D}R^{34D} representing 3- to 6-membered mono-heterocyclic ring which may contain one nitrogen atom and optionally containing one hetero atom selected from nitrogen, oxygen and sulfur atom;
D^{D} is
(1) 1- or 2-membered linker comprising atom(s) selected from carbon, nitrogen, oxygen and sulfur atom, which may contain a double bond or a triple bond and may be substituted by 1 to 4 R^{40D}'s,
(2) 3- to 6-membered linker comprising atoms selected from carbon, nitrogen, oxygen and sulfur, which may contain double bond(s) or triple bond(s) and may be substituted by 1 to 12 R^{40D}'s, wherein R^{40D} substituted on the atom bound to R^{3D}, and R^{42D} which is a substituent of R^{3D} may be taken together to form -(CH₂)_{yD}- wherein y^{D} is 1-4, or
(3) 7- to 10-membered linker comprising atoms selected from carbon, nitrogen, oxygen and sulfur atom, which may contain double bonds or triple bonds and may be substituted by 1 to 20 R^{40D}'s, wherein R^{40D} substituted on the atom binding to R^{3D}, and R^{42D} which is a substituent of R^{3D} may be taken together to form -(CH₂)_{yD}-;
R^{40D} is (a) C1-8 alkyl, (b) C2-8 alkenyl, (c) C2-8 alkynyl, (d) oxo, (e) a halogen atom, (f) CF₃, (g) hydroxy, (h) C1-6 alkoxy, (i) C2-6 alkenyloxy, (j) C2-6 alkynyloxy, (k) OCF₃, (1) -S(O)_{pD}-(C1-6)alkyl, (m) -S(O)_{pD}-(C2-6)alkenyl, (n) -S(O)_{pD}-(C2-6)alkynyl, (o) C2-5 acyl, (p) Cyc9^{D}, (q) C1-4 alkoxy(C1-4)alkoxy, (r) C1-8 alkyl, C2-8 alkenyl or C2-8 alkynyl substituted by 1 or 2 substituents selected from halogen atom, CF₃, OCF₃, hydroxy, cyano, C1-4 alkoxy, -S(O)_{pD}-(C1-6)alkyl, Cyc9^{D} and C1-4 alkoxy(C1-4)alkoxy, or
two R^{40D}'s may be taken together with the atom of a linker to which they bind to form C3-15 mono-, bi- or tri-carbocyclic ring or 3- to 15-membered mono-, bi- or tri-heterocyclic ring containing 1 to 2 hetero atoms selected from O, S, SO₂ and N, wherein the carbocyclic ring and the heterocyclic ring may be substituted by 1 to 3 substituents selected from C1-4 alkyl, C1-4 alkoxy, C2-5 acyl, SO₂(C1-4 alkyl), phenyl and phenyl(C1-4) alkyl;
Cyc9^{D} is C3-6 mono-carbocyclic ring or 3- to 6-membered mono-heterocyclic ring substituted by 1 to 5 R^{41D}'s or unsubstituted;
R^{41D} is C1-4 alkyl, C1-4 alkoxy, C1-4 alkylthio, C1-4 alkoxy(C1-4)alkyl, a halogen atom, CF₃, OCF₃, SCF₃, hydroxy, cyano, formyl, C2-5 acyl, -SO₂-(C1-4)alkyl, - NR^{23D}CO-(C1-4)alkyl, benzyl or oxo;
R^{3D} is
(1) C1-6 alkyl or
(2) C3-15 mono-, bi- or tri-carbocyclic ring or 3- to 15-membered mono-, bi- or tri-heterocyclic ring substituted by 1 to 5 R^{42D}'s or unsubstituted;
R^{42D} is (a) C1-6 alkyl, (b) C1-6 alkoxy, (c) C1-6 alkylthio, (d) a halogen atom, (e) cyano, (f) CF₃, (g) CHF₂, (h) OCF₃, (i) OCHF₂, (j) SCF₃, (k) -NR^{43D}R^{44D}, (1) -SO₂R^{45D}, (m) -NR^{46D}COR^{47D}, (n) hydroxy, (o) oxo, (p) C1-4 alkoxy(C1-4)alkyl, (q) Cyc10^{D}, (r) C1-6 alkylene-Cyc10^{D}, (s) -CO-Cyc10^{D}, (t) -W^{D}-Cyc10^{D}, (u) -(C1-6 alkylene)-W^{D}-Cyc10^{D}, (v) -W^{D}-(C1-6 alkylene)-Cyc10^{D} or (w) -(C1-6 alkylene)-W^{D}-(C1-6 alkylene)-Cyc10^{D};
R^{43D} and R^{44D} are each independently a hydrogen atom or C1-4 alkyl;
R^{45D} is C1-4 alkyl;
R^{46D} is a hydrogen atom or C1-4 alkyl;
R^{47D} is a hydrogen atom or C1-4 alkyl;
Cyc10^{D} is C3-12 mono- or bi-carbocyclic ring or 3- to 12-membered mono- or bi-heterocyclic ring substituted by 1 to 5 substituents selected from the following (a)-(j) or unsubstituted:
(a) C1-4 alkyl, (b) C2-5 acyl, (c) C1-4 alkoxy, (d) a halogen atom, (e) hydroxy, (f) nitro, (g) cyano, (h) amine, (i) CF₃, (j) OCF₃;
W^{D} is -O-, -S(O)_{pD}- or -NR^{48D}-;
R^{48D} is a hydrogen atom or C1-4 alkyl, salts thereof, solvates thereof or prodrugs thereof described in WO03/16254.

Preferably example compounds, salts thereof, solvates thereof or prodrugs thereof described in WO03/16254 are used. Concretely,
(1) (2E)-3-(2-(2-(2,5,7,8-tetramethyl-6-hydroxychroman-2-yl)ethoxy)-4-hydroxymethylphenyl)-2-propenoic acid,
(2) (2E)-3-(2-(2-(naphthalen-2-yl)ethoxy)-4-benzyloxyphenyl)-2-propenoic acid,
(3) 3-(2-(2-(naphthalen-2-yl)ethoxy)-4-phenoxyphenyl)propanoic acid,
(4) 3-(2-(2-(naphthalen-2-yl)ethoxy)-4-hydroxymethylphenyl)propanoic acid,
(5) 3-(2-(2-(naphthalen-2-yl)ethoxy)-4-(1-hydroxy-1-methylethyl)phenyl)propanoic acid,
(6) 3-(2-(((1R)-1-(naphthalen-1-yl)ethyl)carbamoyl)-4-phenoxyphenyl)propanoic acid,
(7) 3-(2-((3-methyl-1-phenylbutyl)carbamoyl)-4-benzyloxyphenyl)propanoic acid,
(8) 3-(2-((3-methyl-1-phenylbutyl)carbamoyl)-4-benzyloxymethylphenyl)propanoic acid,
(9) 3 -(2-((3-methyl-1-phenylbutyl)carbamoyl)-4-cyclopropylmethoxymethylphenyl)propanoic acid,
(10) 2-(2-((4-methyl-2-(naphthalen-1-yl)pentanoyl)amino)-4-hydroxymethylbenzyl)benzoic acid,
(11) 2-(2-((4-methyl-2-(naphthalen-1-yl)pentanoyl)amino)-4-methoxymethylbenzyl)benzoic acid,
(12) 4-(2-((2-(naphthalen-1-yl)propanoyl)amino)-4-hydroxymethylphenyl)butanoic acid,
(13) 4-(2-((2-(naphthalen-1-yl)propanoyl)amino)-4-methoxymethylphenyl)butanoic acid,
(14) 4-(2-((2-(naphthalen-1-yl)propanoyl)amino)-4-benzyloxyphenyl)butanoic acid,
(15) 4-(2-((2-(naphthalen-1-yl)propanoyl)amino)-4-phenoxyphenyl)butanoic acid,
(16) 3-(2-((4-methyl-2-phenylpentanoyl)amino)-4-phenoxyphenyl)propanoic acid,
(17) 4-(2-((4-methyl-2-(naphthalen-1-yl)pentanoyl)amino)-4-phenoxyphenyl)butanoic acid,
(18) 4-(2-((4-methyl-2-phenylpentanoyl)ambo)-4-phenoxyphenyl)butanoic acid,
(19) 4-(2-((2-(naphthalen-1-yl)propanoyl)amino)-4-benzyloxymethylphenyl)butanoic acid,
(20) N-(3,4-difluorophenylsulfonyl)-3-(2-(2-(naphthalen-2-yl)ethoxy)-4-phenoxyphenyl)propanamide,
(21) N-(3,4-difluorophenylsulfonyl)-3-(2-(2-(naphthalen-2-yl)ethoxy)-4-hydroxymethylphenyl)propanamide,
(22) N-(3,4-difluorophenylsulfonyl)-3-(2-(2-(naphthalen-2-yl)ethoxy)-4-(1-hydroxy-1-methylethyl)phenyl)propanamide,
(23) 3 -(2-((3 -methyl-1-phenylbutyl)carbamoyl)-4-methoxymethylphenyl)propanoic acid,
(24) N-(3,4-difluorophenylsulfonyl)-3-(2-((3-methyl-1-phenylbutyl)carbamoyl)-4-methoxymethylphenyl)propanamide,
(25) 3-(2-((3-methyl-1-(3,5-dimethylphenyl)butyl)carbamoyl)-4-benzyloxyphenyl)propanoic acid,
(26) 3-(2-(naphthalen-1-ylmethylcarbamoyl)-4-phenoxyphenyl)propanoic acid,
(27) 3-(2-(1-(naphthalen-2-yl)ethylcarbamoyl)-4-phenoxyphenyl)propanoic acid,
(28) 3-(2-((3-methyl-1-(naphthalen-1-yl)butyl)carbamoyl)-4-phenoxyphenyl)propanoic acid,
(29) 3-(2-(4-methyl-2-phenylpentyl)carbamoyl)-4-phenoxyphenyl)propanoic acid,
(30) 3-(2-((1R)-1-phenylethylcarbamoyl)-4-phenoxyphenyl)propanoic acid,
(31) 4-(2-((1R)-1-(naphthalen-1-yl)ethylcarbamoyl)-4-phenoxyphenyl)butanoic acid,
(32) 3-(2-((1R)-1-(4-methylphenyl)ethylcarbamoyl)-4-phenoxyphenyl)propanoic acid,
(33) 3-(2-(1-(4-fluorophenyl)ethylcarbamoyl)-4-phenoxyphenyl)propanoic acid,
(34) 3-(2-((1R)-1-indan-1-yl)carbamoyl-4-phenoxyphenyl)propanoic acid,
(35) 3-(2-(1-methyl-3-phenylpropyl)carbamoyl-4-phenoxyphenyl)propanoic acid,
(36) 3-(2-((1R)-1-(4-nitrophenyl)ethylcarbamoyl)-4-phenoxyphenyl)propanoic acid,
(37) 3-(2-diphenylmethylcarbamoyl-4-phenoxyphenyl)propanoic acid,
(38) 3-(2-((3-methyl-1-(3,5-dimethylphenyl)butyl)carbamoyl)-4-phenoxyphenyl)propanoic acid,
(39) 3-(2-((1R)-1-1,2,3,4-tetrahydronaphthalen-1-yl)carbamoyl-4-phenoxyphenyl)propanoic acid,
(40) 3-(2-((1R)-1-(1,1'-biphenyl-4-yl)ethylcarbamoyl)-4-phenoxyphenyl)propanoic acid,
(41) 3-(2-(cyano-phenylcarbamoyl)-4-phenoxyphenyl)propanoic acid,
(42) 4-(2-((3-methyl-1-(3,5-dimethylphenyl)butyl)carbamoyl)-4-benzyloxyphenyl)butanoic acid,
(43) 4-(2-((3-methyl-1-(3,5-dimethylphenyl)butyl)carbamoyl)-4-phenoxyphenyl)butanoic acid,
(44) 4-(2-(3-methyl-1-phenylbutyl)carbamoyl)-4-phenoxyphenyl)butanoic acid,
(45) 4-(2-(1-(naphthalen-1-yl)propylcarbamoyl)-4-phenoxyphenyl)butanoic acid,
(46) 4-(2-(1-(naphthalen-1-yl)butylcarbamoyl)-4-phenoxyphenyl)butanoic acid,
(47) 4-(2-((3-methyl-1-(naphthalen-1-yl)butyl)carbamoyl)-4-phenoxyphenyl)butanoic acid,
(48) 4-(2-((3-methyl-1-(4-fluoro-3-methylphenyl)butyl)carbamoyl)-4-phenoxyphenyl)butanoic acid,
(49) 3-(2-(((1R)-3-methyl-1-(3,5-dimethylphenyl)butyl)carbamoyl)-4-phenoxyphenyl)propanoic acid,
(50) 3-(2-((4-(3,5-dimethylphenyl)perhydropyran-4-yl)carbamoyl)-4-phenoxyphenyl)propanoic acid,
(51) 3-(2-((4-(3,5-dimethylphenyl)perhydropyran-4-yl)carbamoyl)-4-benzyloxyphenyl)propanoic acid,
(52) 3-(2-(((1R)-3-methyl-1-(3,5-dimethylphenyl)butyl)carbamoyl)-4-benzyloxyphenyl)propanoic acid,
(53) 3-(2-((4-(naphthalen-1-yl)perhydropyran-4-yl)carbamoyl)-4-phenoxyphenyl)propanoic acid,
(54) 4-(2-((4-(naphthalen-1-yl)perhydropyran-4-yl)carbamoyl)-4-phenoxyphenyl)butanoic acid,
(55) 4-(2-((4-(3,5-dimethylphenyl)perhydropyran-4-yl)carbamoyl)-4-phenoxyphenyl)butanoic acid,
(56) 2-(2-((3-methyl-1-(3,5-dimethylphenyl)butyl)carbamoyl)-4-benzyloxyphenoxy)acetic acid, methyl esters thereof or ethyl esters thereof, or salts thereof are included.

More preferably,
(1) 4-(2-(naphthalen-1-yl)carbonylamino-4-cyanophenyl)butanoic acid,
(2) 3-(6-cyano-1-(2-(naphthalen-1-yl)propionyl)indol-3-yl)propanoic acid,
(3) N-(3,4-difluorophenylsulfonyl)-3-(6-cyano-1-(1-(naphthalen-1-yl)ethylcarbonyl)indol-3-yl)propanamide,
methyl esters thereof or ethyl esters thereof or salts are included.

Moreover, more preferred compounds include
(1) 4-(3 -methyl-1-phenylbutylcarbamoyl)-2-benzofurancarboxylic acid,
(2) 7-(3-methyl-1-phenylbutylcarbamoyl)-2-benzofurancarboxylic acid,
(3) 2-(7-(3-methyl-1-phenylbutylcarbamoyl)indol-1-yl)acetic acid,
(4) 2-(7-(3 -methyl-1-phenylbutylcarbamoyl)indol-3-yl)acetic acid,
(5) 7-(3-methyl-1-phenylbutylcarbamoyl)naphthalenecarboxylic acid,
(6) 2-(7-(3-methyl-1-phenylbutylcarbamoyl)indolin-1-yl)acetic acid,
(7) 3-(7-(3-methyl-1-phenylbutylcarbamoyl)indolin-1-yl)propanoic acid,
(8) 3-(8-(3-methyl-1-phenylbutylcarbamoyl)-1,2,3,4-tetrahydroquinolin-1-yl)propanoic acid,
(9) 2-(8-(3-methyl-1-(3,5-dimethylphenyl)butylcarbamoyl)-1,2,3,4-tetrahydroquinolin-1-yl)acetic acid,
(10) 2-(7-((3 -methyl-1-(3,5-dimethylphenyl)butyl)carbamoyl)indolin-1-yl)acetic acid,
(11) 8-(3-methyl-1-(3,5-dimethylphenyl)butylcarbamoyl)-2-naphthalenecarboxylic acid,
(12) 7-(3-methyl-1-(3,5-dimethylphenyl)butylcarbamoyl)-2-benzofurancarboxylic acid,
(13) 2-(7-(3-methyl-1-(3,5-dimethylphenyl)butylcarbamoyl)benzofuran-2-yl)acetic acid,
(14) 7-((2-(naphthalen-1-yl)acetyl)amino)-2-benzofurancarboxylic acid,
(15) 7-((2-(naphthalen-1-yl)propanoyl)amino)-2-benzofurancarboxylic acid,
(16) 7-((4-methyl-2-(naphthalen-1-yl)pentanoyl)amino)-2-benzofurancarboxylic acid,
(17) 2-(1-(2-(naphthalen-1-yl)propionyl)indol-3-yl)acetic acid,
(18) 2-(2-methyl-1-(2-(naphthalen-1-yl)propionyl)indol-3-yl)acetic acid,
(19) 3-(1-(2-(naphthalen-1-yl)propionyl)indol-3-yl)propanoic acid,
(20) 3-(2-methyl-1-(2-(naphthalen-1-yl)propionyl)indol-3-yl)propanoic acid,
(21) N-(3,4-difluorophenylsulfonyl)-2-(1-(1-(naphthalen-1-yl)ethylcarbonyl)indol-3-yl)acetic acid amide,
(22) N-(3,4-difluorophenylsulfonyl)-2-(2-methyl-1-(1-(naphthalen-1-yl)ethylcarbonyl)indol-3-yl)acetic acid amide,
(23) N-(3,4-difluorophenylsulfonyl)-3-(1-(1-(naphthalen-1-yl)ethylcarbonyl)indol-3-yl)propanamide, methyl esters thereof or ethyl esters thereof, or salts thereof.

Similarly, preferred compounds include
(1) (2E)-3-(2-(6-phenoxyhexyloxy)-4-(imidazol-1-ylmethyl)phenyl)-2-propenoic acid,
(2) 3-(2-(6-phenylhexyloxy)-4-(pyrazol-1-ylmethyl)phenyl)propanoic acid,
(3) N-(3,4-difluorophenylsulfonyl)-3-(2-(5-phenylhexyloxy)-4-(pyrazol-1-ylmethyl)phenyl)propanamide, methyl esters thereof or ethyl esters thereof, or salts thereof. More preferably,

(1) (2E)-3-(2-(2-(naphthalen-2-yl)ethoxy)-4-phenylcarbamoylphenyl)-2-propenoic acid,
(2) 4-(2-((2-(naphthalen-1-yl)propanoyl)amino)-4-phenylphenyl)butanoic acid,
(3) 4-(2-((2-(naphthalen-1-yl)propanoyl)amino)-4-benzylcarbamoylphenyl)butanoic acid,
(4) 4-(2-((2-(naphthalen-1-yl)propanoyl)amino)-4-phenylcarbamoylphenyl)butanoic acid,
(5) 3-(2-((3-methyl-1-phenylbutyl)carbamoyl)-4-cyanophenyl)propanoic acid,
(6) N-(3,4-difluorophenylsulfonyl)-3-(4-cyano-2-((3-methyl-1-phenylbutyl)carbamoyl)phenyl)propanamide,
(7) 3-(2-((3-methyl-1-phenylbutyl)carbamoyl)-4-dibenzylaminophenyl)propanoic acid,
(8) 3-(2-((3-methyl-1-phenylbutyl)carbamoyl)-4-benzylaminophenyl)propanoic acid,
(9) 3-(2-((3-methyl-1-(3,5-dimethylphenyl)butyl)carbamoyl)-4-benzylaminophenyl)propanoic acid,
(10) 3-(2-((3-methyl-1-(3,5-dimethylphenyl)butyl)carbamoyl)-4-(N-benzyl-N-methylamino)phenyl)propanoic acid,
(11) 3-(2-(2-(naphthalen-2-yl)ethoxy)-4-(N-phenylcarbamoyl)phenyl)propanoic acid,
(12) 3-(2-((3-methyl-1-(3,5-dimethylphenyl)butyl)carbamoyl)-4-phenylcarbamoylphenyl)propanoic acid,
methyl esters thereof or ethyl esters thereof, or salts thereof are included.

Moreover, as EP₃ antagonist, compounds represented by formula (E) 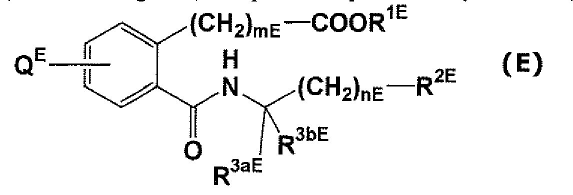 wherein R^{1E} is hydrogen atom or C1-4 alkyl,
R^{2E} is phenyl, naphthyl, benzofuranyl or benzothienyl substituted by 1 or 2 substituent(s) selected from C1-4 alkyl or halogen atom or unsubstituted,
Q^{E} is (i) -CH₂-O-Cycl^{E}, (ii) -CH₂-Cyc2^{E} or (iii) -L-Cyc3,
Cycl^{E} is phenyl or pyridyl substituted by one or two R^{4E}'s or unsubstituted,
Cyc2^{E} is indolyl substituted by one or two R^{4E}'s or unsubstituted,
Cyc3^{E} is phenyl substituted by one or two R^{4E}'s or unsubstituted,
L is -O- or -NH-,
R^{3a} and R^{3b} are each independently hydrogen atom or C1-4 alkyl, or together with the carbon atom to which R^{3aE} and R^{3bE} are attached, form tetrahydro-2H-pyran,
mE is 2 or 3,
nE is 0, 1 or 2,
R^{4E} is C1-4 alkyl, C1-4 alkylthio, halogen atom or cyano, or when Cyc3^{E} is phenyl substituted by two R^{4E}'s, and two R^{4E}'s, together with phenyl, may form 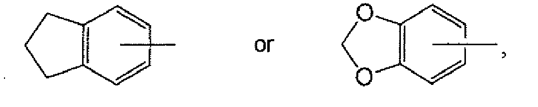 salts thereof, solvates thereof or prodrugs thereof can be used.

Preferably
(1) 3-(4-(2,5-difluorophenoxymethyl)-2-((((1R)-1-(naphthalen-2-yl)ethyl)amino)carbonyl)phenyl)propanoic acid,
(2) 3-(4-(2,5-dichlorophenoxymethyl)-2-(((4-(3-methylphenyl)tetrahydro-2H-pyran-4-yl)amino)carbonyl)phenyl)propanoic acid,
(3) 3-(4-(2-chloro-5-methylphenoxymethyl)-2-(((4-(3-methylphenyl)tetrahydro-2H-pyran-4-yl)amino)carbonyl)phenyl)propanoic acid,
(4) 3-(4-(2-chloro-5-fluorophenoxymethyl)-2-(((4-(3-methylphenyl)tetrahydro-2H-pyran-4-yl)amino)carbonyl)phenyl)propanoic acid,
(5) 3-(4-(2,5-difluorophenoxymethyl)-2-(((4-phenyltetrahydro-2H-pyran-4-yl)amino)carbonyl)phenyl)propanoic acid,
(6) 3-(4-(2,5-dichlorophenoxymethyl)-2-(((4-phenyltetrahydro-2H-pyran-4-yl)amino)carbonyl)phenyl)propanoic acid,
(7) 3-(4-(2-chloro-5-fluorophenoxymethyl)-2-(((4-phenyltetrahydro-2H-pyran-4-yl)amino)carbonyl)phenyl)propanoic acid,
(8) 3-(4-(2,5-difluorophenoxymethyl)-2-((((1R)-3-methyl-1-(3-methylphenyl)butyl)amino)carbonyl)phenyl)propanoic acid,
(9) 3-(4-(3-cyanophenoxymethyl)-2-((((1R)-3-methyl-1-(3-methylphenyl)butyl)amino)carbonyl)phenyl)propanoic acid,
(10) 3-(4-(2,5-dimethylphenoxymethyl)-2-((((1R)-3-methyl-1-(3-methylphenyl)butyl)amino)carbonyl)phenyl)propanoic acid,
(11) 3-(4-(2,5-difluorophenoxymethyl)-2-(((4-(naphthalen-2-yl)tetrahydro-2H-pyran-4-yl)amino)carbonyl)phenyl)propanoic acid,
(12) 3-(4-(2,5-dimethylphenoxymethyl)-2-(((4-(3-methylphenyl)tetrahydro-2H-pyran-4-yl)amino)carbonyl)phenyl)propanoic acid,
(13) 3-(4-(3-cyanophenoxymethyl)-2-(((4-(naphthalen-2-yl)tetrahydro-2H-pyran-4-yl)amino)carbonyl)phenyl)propanoic acid,
(14) 3-(4-(2,5-dimethylphenoxymethyl)-2-(((4-(naphthalen-2-yl)tetrahydro-2H-pyran-4-yl)amino)carbonyl)phenyl)propanoic acid,
(15) 3-(4-(5-fluoroindol-1-ylmethyl)-2-((((1R)-1-(3,5-dimethylphenyl)-3-methylbutyl)amino)carbonyl)phenyl)propanoic acid,
(16) 3-(4-(2,4-dimethylphenoxymethyl)-2-((((1R)-1-(3,5-dimethylphenyl)-3-methylbutyl)amino)carbonyl)phenyl)propanoic acid,
(17) 3-(2-(((4-(naphthalen-2-yl)tetrahydro-2H-pyran-4-yl)amino)carbonyl)-4-phenoxymethylphenyl)propanoic acid,
(18) 3-(2-(((4-(naphthalen-2-yl)tetrahydro-2H-pyran-4-yl)amino)carbonyl)-4-(3-pyridylozymethyl)phenyl)propanoic acid,
(19) 3-(4-(3-chlorophenoxymethyl)-2-(((4-(naphthalen-2-yl)tetrahydro-2H-pyran-4-yl)amino)carbonyl)phenyl)propanoic acid,
(20) 3-(4-(3,4-dimethylphenoxymethyl)-2-((((1R)-1-(3,5-dimethylphenyl)-3-methylbutyl)amino)carbonyl)phenyl)propanoic acid,
(21) 3-(4-(2-chloro-5-fluorophenoxymethyl)-2-((((1R)-1-(3,5-dimethylphenyl)-3-methylbutyl)amino)carbonyl)phenyl)propanoic acid,
(22) 3-(2-((((1R)-1-(3,5-dimethylphenyl)-3-methylbutyl)amino)carbonyl)-4-(3-methylindol-1-ylmethyl)phenyl)propanoic acid,
(23) 3-(4-(2,5-difluorophenoxymethyl)-2-((((1R)-1-(naphthalen-1-yl)ethyl)amino)carbonyl)phenyl)propanoic acid,
(24) 3-(4-(2-fluoro-5-methylphenoxymethyl)-2-((((1R)-1-(naphthalen-1-yl)ethyl)amino)carbonyl)phenyl)propanoic acid,
(25) 3-(2-(((4-(3,5-dimethylphenyl)tetrahydro-2H-pyran-4-yl)amino)carbonyl)-4-(2-fluoro-5-methylphenoxymethyl)phenyl)propanoic acid,
(26) 3-(4-(2-fluoro-5-methylphenoxymethyl)-2-(((4-(3-methylphenyl)tetrahydro-2H-pyran-4-yl)amino)carbonyl)phenyl)propanoic acid,
(27) 3-(2-(((4-(benzofuran-2-yl)tetrahydro-2H-pyran-4-yl)amino)carbonyl)-4-(2,5-difluorophenoxymethyl)phenyl)propanoic acid,
(28) 3-(2-((((1R)-1-(3,5-dimethylphenyl)-3-methylbutyl)amino)carbonyl)-4-(4-fluoro-2-methylphenoxymethyl)phenyl)propanoic acid,
(29) 3-(2-((((1R)-1-(3,5-dimethylphenyl)-3-methylbutyl)amino)carbonyl)-4-((2-methylpyridin-3-yl)oxymethyl)phenyl)propanoic acid,
(30) 3-(2-((((1R)-1-(3,5-dimethylphenyl)-3-methylbutyl)amino)carbonyl)-4-((2-methylpyridin-5-yl)oxymethyl)phenyl)propanoic acid,
(31) 3-(4-(3-fluorophenoxymethyl)-2-(((4-(naphthalen-2-yl)tetrahydro-2H-pyran-4-yl)amino)carbonyl)phenyl)propanoic acid,
(32) 3-(4-(3-methylphenoxymethyl)-2-(((4-(naphthalen-2-yl)tetrahydro-2H-pyran-4-yl)amino)carbonyl)phenyl)propanoic acid,
(33) 3-(4-(2,5-dimethylphenoxymethyl)-2-(((4-(2-phenylethyl)tetrahydro-2H-pyran-4-yl)amino)carbonyl)phenyl)propanoic acid,
(34) 3-(2-(((4-(benzofuran-2-yl)tetrahydro-2H-pyran-4-yl)amino)carbonyl)-4-(2,5-dimethylphenoxymethyl)phenyl)propanoic acid,
(35) 3-(2-(((4-(benzothiophen-2-yl)tetrahydro-2H-pyran-4-yl)amino)carbonyl)-4-(2,5-dimethylphenoxymethyl)phenyl)propanoic acid,
(36) 3-(4-(2,5-difluorophenoxymethyl)-2-(((4-(2-(2-fluorophenyl)ethyl)tetrahydro-2H-pyran-4-yl)amino)carbonyl)phenyl)propanoic acid,
(37) 3-(4-(2,5-dimethylphenoxymethyl)-2-(((4-(2-(2-fluorophenyl)ethyl)tetrahydro-2H-pyran-4-yl)amino)carbonyl)phenyl)propanoic acid,
(38) 3-(4-(2,5-dimethylphenoxymethyl)-2-(((4-(2-(4-fluorophenyl)ethyl)tetrahydro-2H-pyran-4-yl)amino)carbonyl)phenyl)propanoic acid,
(39) 3-(4-(2,5-dimethylphenoxymethyl)-2-(((4-(2-(3-fluorophenyl)ethyl)tetrahydro-2H-pyran-4-yl)amino)carbonyl)phenyl)propanoic acid,
(40) 3-(4-(6-fluoroindol-1-ylmethyl)-2-(((4-(2-phenylethyl)tetrahydro-2H-pyran-4-yl)amino)carbonyl)phenyl)propanoic acid,
(41) 3-(4-(6-fluoroindol-3-ylmethyl)-2-(((4-(2-phenylethyl)tetrahydro-2H-pyran-4-yl)amino)carbonyl)phenyl)propanoic acid,
(42) 3-(4-(3-methylindol-1-ylmethyl)-2-(((4-(2-phenylethyl)tetrahydro-2H-pyran-4-yl)amino)carbonyl)phenyl)propanoic acid,
(43) 3-(4-(3-cyanophenoxymethyl)-2-(((4-(2-phenylethyl)tetrahydro-2H-pyran-4-yl)amino)carbonyl)phenyl)propanoic acid,
(44) 3-(4-(6-fluoroindol-1-ylmethyl)-2-(((4-(naphthalen-2-yl)tetrahydro-2H-pyran-4-yl)amino)carbanyl)phenyl)propanoic acid,
(45) 3-(4-(6-fluoroindol-3-ylmethyl)-2-(((4-(naphthalen-2-yl)tetrahydro-2H-pyran-4-yl)amino)carbonyl)phenyl)propanoic acid,
(46) 3-(4-(3-methylindol-1-ylmethyl)-2-(((4-(naphthalen-2-yl)tetrahydro-2H-pyran-4-yl)amino)carbonyl)phenyl)propanoic acid,
(47) 3-(4-(6-fluoroindol-1-ylmethyl)-2-(((4-(2-(3-fluorophenyl)ethyl)tetrahydro-2H-pyran-4-yl)amino)carbonyl)phenyl)propanoic acid,
(48) 3-(2-(((4-(2-(3-fluorophenyl)ethyl)tetrahydro-2H-pyran-4-yl)amino)carbonyl)-4-(3-methylindol-1-ylmethyl)phenyl)propanoic acid,
(49) 3-(4-(3-cyanophenoxymethyl)-2-(((4-(2-(3-fluorophenyl)ethyl)tetrahydro-2H-pyran-4-yl)amino)carbonyl)phenyl)propanoic acid,
(50) 4-(4-(1,3-dioxaindan-5-yloxy)-2-((((1R)-1-(naphthalen-1-yl)ethyl)amino)carbonyl)phenyl)butanoic acid,
(51) 4-(4-(3 -methylphenoxy)-2-((((1R)-1-(naphthalen-1-yl)ethyl)amino)carbonyl)phenyl)butanoic acid,
(52) 4-(4-(3-cyanophenoxy)-2-((((1R)-1-(naphthalen-1-yl)ethyl)amino)carbonyl)phenyl)butanoic acid,
(53) 4-(4-(3,4-dimethylphenoxy)-2-((((1R)-1-(naphthalen-1-yl)ethyl)amino)carbonyl)phenyl)butanoic acid,
(54) 4-(4-(indan-5-yloxy)-2-((((1R)-1-(naphthalen-1-yl)ethyl)amino)carbonyl)phenyl)butanoic acid,
(55) 4-(4-(3,5-dimethylphenoxy)-2-((((1R)-1-(naphthalen-1-yl)ethyl)amino)carbonyl)phenyl)butanoic acid,
(56) 4-(4-(3-methylthiophenoxy)-2-((((1R)-1-(naphthalen-1-yl)ethyl)amino)carbonyl)phenyl)butanoic acid,
(57) 3-(2-((((1R)-1-(3,5-dimethylphenyl)-3-methylbutyl)amino)carbonyl)-4-(3-fluorophenylamino)phenyl)propanoic acid,
(58) 3-(2-((((1R)-1-(3,5-dimethylphenyl)-3-methylbutyl)amino)carbonyl)-4-(3-methylphenylamino)phenyl)propanoic acid,
(59) 3-(4-(3-cyanophenylamino)-2-((((1R)-1-(3,5-dimethylphenyl)-3-methylbutyl)amino)carbonyl)phenyl)propanoic acid,
(60) 3-(4-(3,5-difluorophenylamino)-2-((((1R)-1-(3,5-dimethylphenyl)-3-methylbutyl)amino)carbonyl)phenyl)propanoic acid,
(61) 3-(2-((((1R)-1-(3,5-dimethylphenyl)-3-methylbutyl)amino)carbonyl)-4-(1,3-dioxaindan-5-ylamino)phenyl)propanoic acid,
(62) 3-(4-(3,5-difluorophenoxy)-2-((((1R)-1-(3,5-dimethylphenyl)-3-methylbutyl)amino)carbonyl)phenyl)propanoic acid,
(63) 3-(4-(3-cyanophenoxy)-2-((((1R)-1-(3,5-dimethylphenyl)-3-methylbutyl)amino)carbonyl)phenyl)propanoic acid,
(64) 4-(4-(3,5-dimethylphenoxy)-2-((((1R)-1-(naphthalen-2-yl)ethyl)amino)carbonyl)phenyl)butanoic acid,
(65) 3-(4-(3,5-dimethylphenoxy)-2-((((1R)-1-(naphthalen-1-yl)ethyl)amino)carbonyl)phenyl)propanoic acid,
(66) 3-(4-(3,5-dimethylphenoxy)-2-((((1R)-1-(naphthalen-2-yl)ethyl) amino)carbonyl)phenyl)propanoic acid,
(67) 3-(4-(3,5-dimethylphenoxy)-2-((((1R)-1-(3-methylphenyl)-3-methylbutyl)amino)carbonyl)phenyl)propanoic acid,
(68) 3-(4-(3-anethylphenylamino)-2-(((4-(naphthalen-2-yl)tetrahydro-2H-pyran-4-yl)amino)carbonyl)phenyl)propanoic acid,
(69) 4-(4-(3-fluorophenylamino)-2-((((1R)-1-(naphthalen-1-yl)ethyl)amino)carbonyl)phenyl)butanoic acid,
(70) 4-(4-(3 -methylphenylamino)-2-((((1R)-1-(naphthalen-1-yl)ethyl)amino)carbonyl)phenyl)butanoic acid,
(71) 4-(4-(3,5-difluorophenylamino)-2-((((1R)-1-(naphthalen-1-yl)ethyl)amino)carbonyl)phenyl)butanoic acid,
(72) 4-(4-(1,3-dioxaindan-5-ylamino)-2-((((1R)-1-(naphthalen-1-yl)ethyl)amino)carbonyl)phenyl)butanoic acid,
(73) 4-(4-(3-cyanophenylamino)-2-((((1R)-1-(naphthalen-1-yl)ethyl)amino)carbonyl)phenyl)butanoic acid,
(74) 3-(4-(3,5-dimethylphenylamino)-2-((((1R)-1-(naphthalen-1-yl)ethyl)amino)carbonyl)phenyl)propanoic acid,
(75) 3-(4-(3,5-dimethylphenylamino)-2-((((1R)-1-(naphthalen-2-yl)ethyl)amino)carbonyl)phenyl)propanoic acid,
(76) 3-(4-(3,5-dimethylphenylamino)-2-(((4-(3,5-dimethylphenyl)tetrahydro-2H-pyran-4-yl)amino)carbonyl)phenyl)propanoic acid,
(77) 3-(4-(3,5-dimethylphenylamino)-2-(((4-(3-methylphenyl)tetrahydro-2H-pyran-4-yl)amino)carbonyl)phenyl)propanoic acid,
(78) 3-(4-(3,5-dimethylphenoxy)-2-(((4-(3-methylphenyl)tetrahydro-2H-pyran-4-yl)amino)carbonyl)phenyl)propanoic acid,
(79) 3-(2-(((4-(benzofuran-2-yl)telrahydro-2H-pyran-4-yl)amino)carbonyl)-4-(3,5-dimethylphenoxy)phenyl)propanoic acid,
(80) 3-(2-(((4-(benzofuran-2-yl)tetrahydro-2H-pyran-4-yl)amino)carbonyl)-4-(3,5-dimethylphenylamino)phenyl)propanoic acid,
(81) 3-(4-(3,5-dimethylphenoxy)-2-(((4-(2-phenylethyl)tetrahydro-2H-pyran-4-yl)amino)carbonyl)phenyl)propanoic acid,
(82) 3-(4-(3,5-dimethylphenoxy)-2-(((4-(3,5-dimethylphenyl)tetrahydro-2H-pyran-4-yl)amino)carbonyl)phenyl)propanoic acid,
(83) 3-(4-(3,5-dimethylphenylamino)-2-(((4-(2-phenylethyl)tetrahydro-2H-pyran-4-yl)amino)carbonyl)phenyl)propanoic acid,
(84) 3-(2-(((4-(3,5-dimethylphenyl)tetrahydro-2H-pyran-4-yl)amino)carbonyl)-4-(3-methylphenylamino)phenyl)propanoic acid,
(85) 3-(2-(((4-(benzothiophen-2-yl)tetrahydro-2H-pyran-4-yl)amino)carbonyl)-4-(3,5-dimethylphenylamino)phenyl)propanoic acid,
(86) 3-(4-(3,5-dimethylphenoxy)-2-(((4-(2-(4-fluorophenyl)ethyl)tetrahydro-2H-pyran-4-yl)amino)carbonyl)phenyl)propanoic acid,
(87) 3-(4-(3,5-dimethylphenylamino)-2-(((4-(2-(4-fluorophenyl)ethyl)tetrahydro-2H-pyran-4-yl)amino)carbonyl)phenyl)propanoic acid,
(88) 3-(4-(3,5-dimethylphenylamino)-2-(((4-(2-(2-fluorophenyl)ethyl)tetrahydro-2H-pyran-4-yl)amino)carbonyl)phenyl)propanoic acid,
(89) 3-(4-(3,5-dimethylphenoxy)-2-(((4-(2-(2-fluorophenyl)ethyl)tetrahydro-2H-pyran-4-yl)amino)carbonyl)phenyl)propanoic acid, or
(90) 3-(4-(3,5-dimethylphenoxy)-2-(((4-(2-(3-fluorophenyl)ethyl)tetrahydro-2H-pyran-4-yl)amino)carbonyl)phenyl)propanoic acid,
   salts thereof, solvates thereof, or prodrugs thereof can be used.

Specifically,
(1) 3-(2-((((1R)-1-(3,5-dimethylphenyl)-3-methylbutyl)amino)carbonyl)-4-(5-fluoro-2-methylphenoxymethyl)phenyl)propanoic acid,
(2) 3-(2-(((4-(benzothiophen-2-yl)tetrahydro-2H-pyran-4-yl)amino)carbonyl)-4-(2,5-difluorophenoxymethyl)phenyl)propanoic acid,
(3) 3-(4-(2-fluoro-5-methylphenoxymethyl)-2-((((1R)-3-methyl-1-(3-methylphenyl)butyl)amino)carbonyl)phenyl)propanoic acid,
(4) 3-(4-(2,5-difluorophenoxymethyl)-2-(((4-(2-(3-fluorophenyl)ethyl)tetrahydro-2H-pyran-4-yl)amino)carbonyl)phenyl)propanoic acid,
(5) 3-(4-(3,5-dimethylphenylamino)-2-(((4-(naphthalen-2-yl)tetrahydro-2H-pyran-4-yl)amino)carbonyl)phenyl)propanoic acid,
(6) 3-(2-(((4-(benzothiophen-2-yl)tetrahydro-2H-pyran-4-yl)amino)carbonyl)-4-(3,5-dimethylphenoxy)phenyl)propanoic acid,
(7) 3-(4-(2,5-difluorophenoxymethyl)-2-(((4-(2-phenylethyl)tetrahydro-2H-pyran-4-yl)amino)carbonyl)phenyl)propanoic acid,
(8) 3-(2-((((1R)-1-(3,5-dimethylphenyl)-3-methylbutyl)amino)carbonyl)-4-(3-pyridyloxymethyl)phenyl)propanoic acid,
(9) 3-(4-(2-fluoro-5-methylphenoxymethyl)-2-((((1R)-1-(3,5-dimethylphenyl)-3-methylbutyl)amino)carbonyl)phenyl)propanoic acid,
(10) 3-(4-(3,5-dimethylphenylamino)-2-((((1R)-1-(3,5-dimethylphenyl)-3-methylbutyl)amino)carbonyl)phenyl)propanoic acid,
(11) 3-(4-(6-fluoroindol-1-ylmethyl)-2-((((1R)-1-(3,5-dimethylphenyl)-3-methylbutyl)amino)carbonyl)phenyl)propanoic acid,
(12) 3-(4-(3,5-dimethylphenoxy)-2-(((4-(naphthalen-2-yl)tetrahydro-2H-pyran-4-yl)amino)carbonyl)phenyl)propanoic acid,
(13) 4-(4-(3,5-dimethylphenylamino)-2-((((1R)-1-(naphthalen-1-yl)ethyl)amino)carbonyl)phenyl)butanoic acid,
(14) 3-(4-(2-chloro-5-methylphenoxymethyl)-2-((((1R)-3-methyl-1-(3-methylphenyl)butyl)amino)carbonyl)phenyl)propanoic acid,
(15) 3-(4-(2,5-difluorophenoxymethyl)-2-(((4-(2-(4-fluorophenyl)ethyl)tetrahydro-2H-pyran-4-yl)amino)carbonyl)phenyl)propanoic acid,
(16) 3-(4-(3,5-dimethylphenylamino)-2-((((1R)-1-(3-methylphenyl)-3-metbylbutyl)arnino)carbonyl)phenyl)propanoic acid,
(17) 3-(4-(2-fluoro-5-methylphenoxymethyl)-2-(((4-(naphthalen-2-yl)tetrahydro-2H-pyran-4-yl)amino)carbonyl)phenyl)propanoic acid,
(18) 3-(4-(3,5-dimethylphenoxy)-2-((((1R)-1-(3,5-dimethylphenyl)-3-methylbutyl)amino)carbonyl)phenyl)propanoic acid, or
(19) 3-(4-(3,5-dimethylphenylamino)-2-(((4-(2-(3-fluorophenyl)ethyl)tetrahydro-2H-pyran-4-yl)amino)carbonyl)phenyl)propanoic acid,
salts thereof, solvates thereof, or prodrugs thereof can be used as preferably.

### Salts:

The salt used in the present invention is preferably non-toxic and water-soluble. The suitable salt means, for example, salt of alkaline metal (potassium, sodium, *etc*.), salt of alkaline earth metal (calcium, magnesium, *etc*.), ammonium salt, pharmaceutically acceptable salt of organic amine (tetramethylammonium, triethylamine, methylamine, dimethylamine, cyclopentylamine, benzylamine, phenethylamine, piperidine, monoethanolamine, diethanolamine, tris(hydroxymethyl)methylamine, lysine, arginine, N-methyl-D-glucamine, *etc*.).

The acid addition salt is preferably non-toxic and water-soluble. The suitable acid addition salt means, for example, inorganic acid salt (hydrochloride, hydrobromate, sulfate, phosphate, nitrate, *etc.*), or organic acid salt (acetate, trifluoroacetate, lactate, tartrate, oxalate, fumarate, maleate, citrate, benzoate, methane sulfonate, ethane sulfonate, benzene sulfonate, toluene sulfonate, isethionate, glucuronate, gluconate, *etc*.), *etc*.

The compound used in the present invention and the salt thereof may be converted hydrate by known methods.

The combination of EP₁ antagonist and EP₃ antagonist of the present invention can improve urinary storage disorder, concretely, anomaly of urine retaining ability of bladder, decreasing bladder compliance, hypertonic detrusor muscle and hypersensitive bladder. Namely, the present invention can improve the storage capacity of the bladder, that is, the present invention can increase the storage amount of the bladder. Moreover, the present invention can improve bladder compliance and hypertonic detrusor muscle, and has an effect to normalize bladder afferent. Therefore, it has an effect to prevent and/or treatment for urgency of urination, bladder pain, frequent urination, night urination or urine incontinence.

The effects become visible by a combination of EP₁ antagonist and EP₃ antagonist, namely by using EP₁ antagonist in combination with EP₃ antagonist or using one preparation comprising them. Moreover, EP₁ antagonist and EP₃ antagonist may be the same compound, namely the compound having antagonism to EP₁ and EP₃. In the case of using EP₁ antagonist in combination with EP₃ antagonist or using one preparation comprising them, the mass ratio of EP₁ antagonist and EP₃ antagonist is not limited.

Moreover, even when EP₁ antagonist of low dose, in a word, the amount from which an enough effect is not seen in a single administering and EP₃ antagonist of low dose, in a word, the amount from which an enough effect is not seen in a single administering are combined, the effect becomes visible. Such a fact is not easily expected.

### Processes for the preparation of the compound used in the present invention:

The EP₁ antagonists used in the present invention can be prepared by methods described in the specification of above-described (1)-(39). The EP₃ antagonists used in the present invention can be prepared by methods described in the specification of above-described (40)-(47) and the specification of international application No. PCT/JP2004/001262.

### Toxicity:

It has been confirmed that the compounds of the present invention have low toxicity and are sufficiently safe for use as pharmaceutical preparations.

### INDUSTRIAL APPLICABILITY

### Application to pharmaceuticals:

A combination of EP₁ antagonist and EP₃ antagonist or a compound having antagonism to EP₁ and EP₃ has effect of improving urine retaining ability, improving bladder compliance, relieving hypertonic detrusor muscle and normalizing bladder perception. Moreover, the combination of EP₁ antagonist and EP₃ antagonist or the compound having antagonism to EP₁ and EP₃ is useful in preventing and/or treating urinary tract diseases with symptoms such as urgency of urination, bladder pain, frequent urination, night urination or urine incontinence.

The combination of EP₁ antagonist and EP₃ antagonist or the compound having antagonism to EP₁ and EP₃ may be administered as a combined preparation by combining with other medicaments for the purpose of
1) supplementing and/or enhancing of prevention and/or treatment effect,
2) improvement in pharmacokinetics and absorption and reduction of dose, and/or
3) reduction of side effect.

The combined preparation of the combination of EP₁ antagonist and EP₃ antagonist or the compound having antagonism to EP₁ and EP₃ of the present invention with other medicaments may be administered in a form of a compounded agent in which both components are compounded in a preparation or may be in a form in which they are administered by means of separate preparations. The case of administration by means of separate preparations includes a simultaneous administration and administrations with time difference. In the case of administrations with time difference, the combination of EP₁ antagonist and EP₃ antagonist or the compound having antagonism to EP₁ and EP₃ of the present invention may be firstly administered followed by administering the other medicament, or the other medicament may be administered firstly followed by administering the combination of EP₁ antagonist and EP₃ antagonist or the compound having antagonism to EP₁ and EP₃ of the present invention. In addition, the case of the combination of the present invention, after previously administering one of EP₁ antagonist and EP₃ antagonist, and administering other medicines, the other of EP₁ antagonist and EP₃ antagonist may be administrated. Methods for each of the administration may be the same or different.

As other medicaments for supplementing and/or enhancing the treatment effect for urgency of urination, bladder pain, frequent urination, night urination or urine incontinence of the combination of EP₁ antagonist and EP₃ antagonist or the compound having antagonism to EP₁ and EP₃ of the present invention, for example, anticholinergic drugs, tricyclic antidepressants, α₁ agonists, α₁ antagonists, GABA agonist, antidiuretics, antiandrogen, progestational hormones, NK₁ antagonists, β₃ agonists, P2X antagonist, potassium channel openers, LPA, capsaicin (resiniferatoxin), muscarinic (M1, M3) antagonists, 5-HT reuptake inhibitors, 5-HT_{1A} antagonists, ACh antagonists, Ca channel antagonist etc are included.

Examples of anticholinergic drugs include oxybutynin hydrochloride, bethanechol chloride, propiverine hydrochloride, propantheline bromide, methylbenactyzium bromide, butylscopolamine bromide, tolterodine tartrate, trospium chloride, Z-338, K-112166-04, ONO-8025, darifenacin, YM-905 and the like.

Example of muscarinic antagonists include YM905, ONO-8025 and the like.

The ratio by mass of the combination of EP₁ antagonist and EP₃ antagonist or the compound having antagonism to EP₁ and EP₃ of the present invention to other medicaments is not particularly limited.

Two or more of other medicaments optionally selected can be used in combination.

Other medicaments to be used for complementing and/or enhancing the preventive and/or therapeutic effects of the combination of EP₁ antagonist and EP₃ antagonist or the compound having antagonism to EP₁ and EP₃ of the present invention include not only those which have been found out hitherto based on the above-described mechanism but also those which will found out in future.

To employ the combination of EP₁ antagonist and EP₃ antagonist or the compound having antagonism to EP₁ and EP₃ of the present invention for the above-described purposes, they are usually administered systemically or topically, and orally or parenterally.

The doses to be administered are determined depending upon, for example, age, body weight, symptom, the desired therapeutic effect, the route of administration, and the duration of the treatment. In the human adult, the doses per person are generally from 1 mg to 1000 mg, by oral administration, up to several times per day, and from 0.1 mg to 100 mg, by parenteral administration (preferably intravenous administration), up to several times per day, or continuous administration from 1 to 24 hours per day from vein.

As mentioned above, the doses to be used depend upon various conditions. Therefore, there are cases in which doses lower than or greater than the ranges specified above may be used.

The combination of EP₁ antagonist and EP₃ antagonist, the compound having antagonism to EP₁ and EP₃ or concomitant medication combined the compound of the combination of EP₁ antagonist and EP₃ antagonist or the compound having antagonism to EP₁ and EP₃ with other medicament may be administered in the composition of, for example, solid compositions or liquid compositions, each for oral administration, or injections, external use or suppositories each for parenteral administration.

Examples of the solid preparations for internal use for oral administration include tablets, pills, capsules, powders, granules and the like. The capsules include hard capsules and soft capsules.

Such a solid preparation for internal use is prepared by a formulation method commonly employed by using one or two or more active substances either as it is or as a mixture with an excipient (lactose, mannitol, glucose, microcrystalline cellulose, starch, *etc*.), a binder (hydroxypropylcellulose, polyvinylpyrrolidone, magnesium metasilicate aluminate, *etc*.), a disintegrating agent (calcium cellulose glycolate, *etc*.), a lubricant (magnesium stearate, *etc*.), a stabilizer and a dissolution aid (glutamic acid, aspartic acid, *etc.).* If necessary, it may be coated with a coating agent (sucrose, gelatin, hydroxypropylcellulose, hydroxypropylmethylcellulose phthalate, *etc*.). It may be coated with two or more layers. Moreover, capsules made of an absorbable material such as gelatin are involved in the scope thereof

Liquid forms for oral administration include pharmaceutically acceptable solutions, suspensions and emulsions, syrups and elixirs. In such forms, one or more of the active compound(s) may be dissolved, suspended or emulized into diluent(s) commonly used in the art (such as purified water, ethanol or a mixture thereof). Besides such liquid forms may also comprise some additives, such as wetting agents, suspending agents, emulsifying agents, sweetening agents, flavoring agents, aroma, preservative or buffering agent.

Injections for parenteral administration include sterile aqueous, suspensions, emulsions and solid forms which are dissolved or suspended into solvent(s) for injection immediately before use. In injections, one or more of the active compound(s) may be dissolved, suspended or emulized into solvent(s). The solvents may include distilled water for injection, physiological salt solution, vegetable oil, propylene glycol, polyethylene glycol, alcohol, e.g. ethanol, or a mixture thereof Injections may comprise some additives, such as stabilizing agents, solution adjuvants (such as glutamic acid, aspartic acid or POLYSORBATE80 (registered trade mark)), suspending agents, emulsifying agents, soothing agent, buffering agents, preservative.
They may be sterilized at a final step, or may be prepared by an aseptic manipulation .
They may also be manufactured in the form of sterile solid forms, for example, freeze-dried products, which may be dissolved in sterile water or some other sterile diluent(s) for injection immediately before use.

In the parenteral administration, formulation of external use include, for example, ointment, ger, cream, poultice, patch, liniment, atomized agent, inhalation, spray, eye drops and nasal spray, etc. They includes one or more of the active compound(s) and be prepared by known method or usual method.

The other compositions for parenteral administration include suppositories for intrarectal administration and pessaries for vaginal administration which comprise one or more of the active substance(s) and may be prepared by methods known *per se*.

### Advantageous effect of the invention

The combination of an EP₁ antagonist with an EP₃ antagonist is useful in preventing and/or treating urinary tract diseases with symptoms such as urgency of urination, bladder pain, frequent urination or urine incontinence, because of showing effect of improving urine retaining ability, improving bladder compliance, relieving hypertonic detrusor muscle and normalizing bladder perception.

### Brief Description of the Drawings

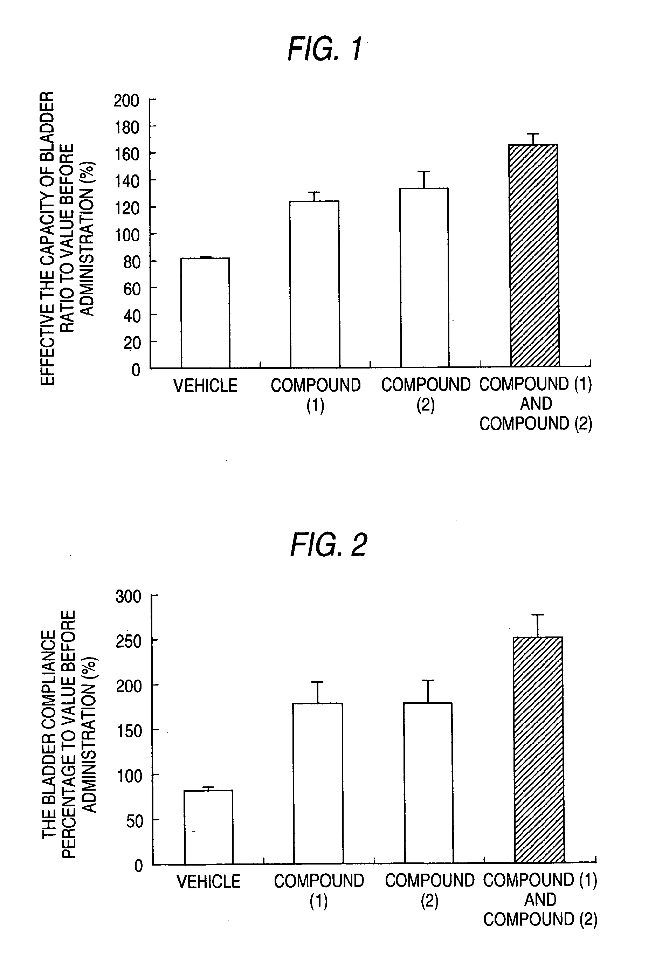

Fig. 1 shows the percentage of the value of the effective bladder capacity in the case that 3 mg/kg of the compound (1); 3-methyl-4-[6-[n-isobutyl-n-(4-methyl-2-thiazolylsulfonyl)amino]indan-5-yloxymethyl]cinnamic acid sodium salt and 1 mg/kg of the compound (2); N-(3,4-difluorophenylsulfonyl)-3-(2-(2-(naphthalen-2-yl)ethoxy)-4-(3-cyanphenoxymethyl)phenyl)propanamide sodium salt are administered singly or simultaneously in combination, to the value thereof before the administration of the test compounds.
Fig. 2 shows the percentage of the value of the bladder compliance in the case that 3 mg/kg of the compound (1) and 1 mg/kg of the compound (2) are administered singly or simultaneously in combination, to the value thereof before the administration of the test compounds.

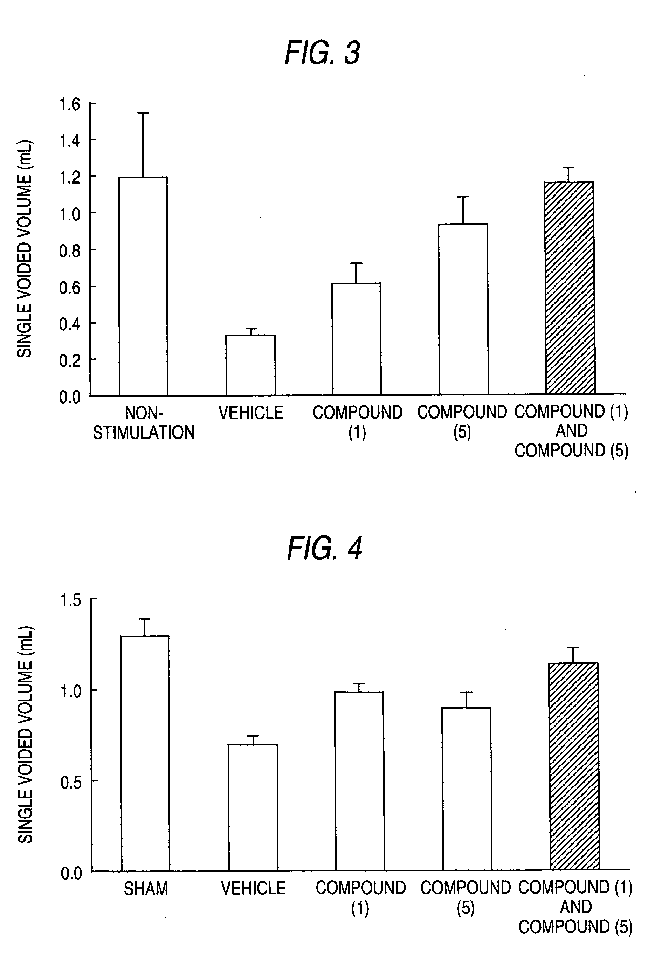

Fig. 3 shows the effect on single voided volume in an animal model of overactive bladder induced by sulprostone, in the case that 10 mg/kg of the compound (1) and 30 mg/kg of the compound (5); 3-[4-[(2,5-dimethylphenoxy)methyl]-2-({[(1R)-1-(3,5-diethylphenyl)-3-methylbutyl]amino}carbonyl)phenyl]propanoic acid are administered singly or simultaneously in combination.
Fig. 4 shows the effect on single voided volume in an animal model of overactive bladder induced by acetic acid, in the case that 10 mg/kg of the compound (1) and 30 mg/kg of the compound (5) are administered singly or simultaneously in combination.

### Best Mode for Carrying Out the Invention

The present invention is detailed by the following experiments, preparation examples and tests. However, that the present invention is not limited thereto.

### Example 1

Effect on the amelioration of urination function during the perfusion of PGE₂ solution in bladder

### Catheter indwelling:

Female SD-IGS rats (around 9 weeks old) were anesthetized with sodium pentobarbital (40 mg/kg, i.p.). After median incision of the hypogastrium, the top of bladder was incised. A catheter for use in cystometry was filled with physiological saline and then was inserted through the top hole into bladder. The other end of the catheter was fixed subcutaneously in the dorsal part. Viccillin S500 (Meiji Seika Kaisha, Ltd.; 10 mg titers/0.1 mL distilled water/rat) was injected into the buttock muscle. Then, the rats were fed for 6 days or more, and subsequently subjected to cystometry.

### Preparation of cystometry:

After indwelling of the catheter and feeding for 6 days or more, the rats were anesthetized with ether. A catheter for use in pharmaceutical administration was preliminarily filled with physiological saline and indwelled in the common carotid vein, while the other end was drawn out of the dorsal part. The tip of the bladder catheter was connected through a three-way valve to a pressure transducer. Using an amp recorder of strain pressure, inner bladder pressure was recorded. Another end of the three-way valve was connected to a syringe for injection into bladder, which was mounted on an infusion pump, while the other end was connected to an extension tube filled with physiological saline, for use in discharging residual urine. The rats after the treatment were left until they awoke from anesthesia.

### Experimental method:

Physiological saline containing 60 µmol/L prostaglandin E₂ (a solution with a final ethanol concentration of 0.1%) was perfused at a rate of 2.85 mL/h for 3 hours in the bladders of such rats thus treated. It was then confirmed that the ratio of the change of effective bladder capacity as measured twice was within 20%. Subsequently, a test compound was administered from the venous catheter.

The parameter values of effective bladder capacity and bladder compliance before and after the administration were read, to express the parameter values in percentage to the parameter values before the administration.

As the test compounds, there were used 3 mg/kg of 3-methyl-4-[6-[N-isobutyl-N-(4-methyl-2-thiazolylsulfonyl)amino]indan-5-yloxymethyl]cinnamic acid sodium salt as EP₁ antagonist (compound (1) : a compound described in WO 02/72564) and 1 mg/kg of N-(3,4-difluorophenylsulfonyl)-3-(2-(2-(naphthalen-2-yl)ethoxy)-4-(3-cyanphenoxymethyl)phenyl)propanamide sodium salt as EP₃ antagonist (compound (2) : a sodium salt of N-(3,4-difluoraphenylsulfonyl)-3-(2-(2-(naphthalen-2-yl)ethoxy)-4-(3-cyanphenoxymethyl)phenyl)propanamide described in WO 03/16254). The compounds were administered singly or simultaneously in combination.

Consequently, the administration of the compounds (1) and (2) in combination enhanced the effect of ameliorating bladder capacity and bladder compliance, in comparison with the administration of each of the compounds alone (Figs. 1 and 2).

### Example 2

### Effect of suppressing resected urinary detrusor contraction induced by PGE₂

Under anesthesia with pentobarbital (50 mg/kg, i.p.), male SD-IGS rats are exsanguinated to death via cutting the carotid arteries. The abdominal part was incised to resect the bladder, which was then immersed in ice-cold Krebs buffer saturated with a mix gas (95% oxygen and 5% carbon dioxide), to prepare a reed-shaped specimen by cutting the bladder body along the longitudinal direction. The prepared bladder specimen was suspended in a Krebs buffer (of 5 mL at 37°C) purged with the mix gas, under a load of about 1 g.

Using a magnus apparatus system (Iwashiya Kishimoto Medical Instruments) equipped with an isometric transducer (UFER UM-203) and an amp (UFER AP-5), the tension of the specimen was recorded through a data recovery system (NR-1000; KEYENCE CORPORATION) with a computer machine.

One hour or more after the start of the suspension of the specimen, potassium chloride (at a final concentration of 100 mmol/L) was added for the observation of the maximum contraction.

PGE₂ (0.3 nmol/L to 30 µmol/L) was cumulatively added to observe the PGE₂ reaction before the treatment with test compounds. After the specimen was washed with the Krebs buffer, the test compounds were treated and PGE₂ was cumulatively added 10 minutes later. The PGE₂ reaction after the treatment with the test compounds was assayed.

The change of the tension via the PGE₂ addition at each concentration was read to be evaluated on the basis of the percentage to the maximum reaction of PGE₂ before the treatment with the pharmaceutical agents.

As the test compounds, there were used 3-methyl-4-[6-[N-isobutyl-N-(4-methyl-2-thiazolylsulfonyl)amino]indan-5-yloxymethyl]cinnamic acid (compound (3) : a compound described in WO 02/72564) as EP₁ antagonist and 3-(2-(((1R)-3-methyl-1-(3,5-dimethylphenyl)butyl)carbamoyl)-4-(2,5-difluorophenoxymethyl)phenyl)propanoic acid (compound (4) : a compound described in WO 03/16254) as EP₃ antagonist. The compounds were administered singly or simultaneously in combination.

Consequently, the use of the compounds (3) and (4) in combination significantly suppressed the PGE₂-induced contraction of urinary detrusor. The present experiment revealed that the use of the compounds (3) and (4) in combination ameliorated excessive contraction of urinary detrusor.

### Example 3

### Effect of suppressing overactive bladder induced by sulprostone

One hour before sulprostone administration, test compounds were orally administered. Thereafter, sulprostone (0.2 mg/kg) was subcutaneously administered. The weight of excreted urine was recorded on a hard disk with a data collection system (NR-1000; KEYENCE CORPORATION), after animals were placed in a metabolic cage equipped with an urine measurement apparatus (Neuroscience). The weight of the excreted urine in 3 hours after sulprostone administration was measured. The frequency of urination and single voided volume were used as assessment items.

As the test compounds, there were used 3-methyl-4-[6-[N-isobutyl-N-(4-methyl-2-thiazolylsulfonyl)amino]indan-5-yloxymethyl]cinnamic acid sodium salt (compound (1) : a compound described in WO 02/72564) as EP₁ antagonist, and 3-[4-[(2,5-dimethylphenoxy)methyl]-2-({[(1R)-1-(3,5-diethylphenyl)-3-methylbutyl]amino}carbonyl)phenyl]propanoic acid (compound (5) : a compound described in WO 03/16254) as EP₃ antagonist. These compounds were administered singly or simultaneously in combination.

Consequently, the administration of the compound (1) and the compound (5) in combination enhanced the improving effect on the decrease of single voided volume due to sulprostone, in comparison with the single administration of each of the compounds (Fig. 3). It is known that sulprostone has a selective agonist action for EP₁ and EP₃, and EP₁ and EP₃ are involved in the occurrence of urinary tract diseases. The experiment revealed that the combined use of the EP₁ and EP₃ antagonists enhanced the action of ameliorating such symptoms of urinary tract diseases. Additionally, the improving action on the increase of the frequency of urination was also enhanced.

### Example 4

### Effect of suppressing overactive bladder induced by acetic acid

Under pentobarbital anesthesia (50 mg/kg, i.p.), animals were fixed on their supine positions. The hypogastrium was incised along the median line to expose the bladder. Then, 0.5 mL of physiological saline containing acetic acid to 1% was injected into the bladder, and subsequently, the incision was sutured. To a non-stimulation group, 0.5 mL of physiological saline was injected in place of the acetic acid solution. Two days later, test compounds were orally administered and excreted urine weight was measured with the metabolic cage. The frequency of urination and the single voided volume over 6 hours after 30 minutes from the administration of the pharmaceutical agents were used as assessment items.

The same compounds (1) and (5) as in Example 3 were used as the test compounds.

Consequently, the combined used of the compounds (1) and (5) enhanced the improving effect on the decrease of single voided volume as induced by acetic acid, in comparison with the single administration of each of the compounds (Fig. 4).
Additionally, the improving action on the increase of the frequency of urination was also enhanced.

### Preparation Example 1:

The following components were admixed in a conventional method, punched out to give 1,000,000 tablets each containing 10 mg of active ingredient.

| | |
|---|---|
| 3-methyl-4-[6-[N-isobutyl-N-(4-methyl-2-thiazolylsulfonyl)amino]indan-5-yloxymethyl]cinnamic acid sodium salt | 7.5kg |
| ·N-(3,4-difluorophenylsulfonyl)-3-(2-(2-(naphthalen-2-yl)ethoxy)-4-(3-cyanophenoxymethyl)phenyl)propanamide sodium salt | 2.5kg |
| · calcium carboxymethylcellulose (disintegrant) | 2kg |
| · magnesium stearate (lubricant) | 1kg |
| · microcrystalline cellulose | 87kg |

### Preparation Example 2:

The following components were admixed in a conventional method, and the solution was filtrated by dust filter, placed at 5 ml into amples and heat sterilized by autoclave to thereby obtain 1,000,000 amples each containing 20 mg of the active ingredient.

| | |
|---|---|
| ·3-methyl-4-[6-[N-isobutyl-N-(4-methyl-2-thiazolylsulfonyl)amino]indan-5-yloxymethyl]cinnamic acid sodium salt | 15kg |
| N-(3,4-difluorophenylsulfonyl)-3-(2-(2-(naphthalen-2-yl)ethoxy)-4-(3-eyanophenoxymethyl)phenyl)propanamide sodium salt | 5kg |
| · mannitol | 2kg |
| · distilled water | 5kl |

## Claims

1. An agent for preventing and/or treating urinary tract disease comprising a combination of EP₁ antagonist and EP₃ antagonist.

2. The agent for preventing and/or treating urinary tract disease according to claim 1, wherein the urinary tract disease is lower urinary tract disorder.

3. The agent for preventing and/or treating urinary tract disease according to claim 1, wherein the urinary tract disease is urinary storage disorder.

4. The agent for preventing and/or treating urinary tract disease according to claim 3, wherein the urinary storage disorder is overactive bladder.

5. The agent for preventing and/or treating urinary tract disease according to claim 4, wherein the overactive bladder is urgency of urination, bladder pain or urine incontinence.

6. The agent for preventing and/or treating urinary tract disease according to claim 4, wherein the overactive bladder is frequent urination.

7. The agent for preventing and/or treating urinary tract disease according to claim 5, wherein the urine incontinence is urgency incontinence, stress urinary incontinence, overflow incontinence, psychogenic incontinence or complex incontinence.

8. The agent for preventing and/or treating urinary tract disease according to claim 1, which is an agent for improving urine retaining ability.

9. The agent for preventing and/or treating urinary tract disease according to claim 1, which is an agent for improving bladder compliance.

10. The agent for preventing and/or treating urinary tract disease according to claim 1, which is an agent for relieving hypertonic detrusor muscle.

11. The agent for preventing and/or treating urinary tract disease according to claim 1, wherein the EP₁ antagonist is a compound selected from a group consisting of a compound represented by formula (A) 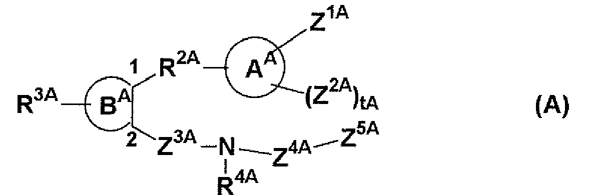 wherein 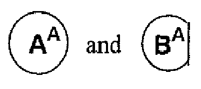 are each independently C5-15 carbocyclic ring or 5-to 7-membered heterocyclic ring having 1 or 2 oxygen, sulfur or nitrogen atoms;
Z^{1A} is a group represented by -COR^{1A}, -C1-4 alkylene-COR^{1A}, -CH=CH-COR^{1A}, -C≡C-COR^{1A}, -O-C1-3 alkylene-COR^{1A} wherein R^{1A} is hydroxy, C1-4 alkoxy or a group represented by formula NR^{6A}R^{7A} wherein R^{6A} and R^{7A} are independently hydrogen atom or C1-4 alkyl or -C1-5 alkylene-OH;
Z^{2A} is a hydrogen atom, C1-4 alkyl, C1-4 alkoxy, nitro, halogen, trifluoromethyl, trifluoromethoxy, hydorxy or a group represented by formula COR^{1A} wherein R^{1A} has the same meaning as described above;
Z^{3A} is a single bond or C1-4 alkylene;
Z^{4A} is SO₂ or CO;
Z^{5A} is (1) C1-8 alkyl, C2-8 alkenyl, C2-8 alkynyl, (2) phenyl, C3-7 cycloalkyl, 5-to 7-membered heterocyclic ring having 1 or 2 oxygen, sulfur or nitrogen atoms, (3) C1-4 alkyl, C2-4 alkenyl or C2-4 alkynyl substituted by phenyl or C3-7 cycloalkyl wherein phenyl, C3-7 cycloalkyl and 5- to 7-membered heterocyclic ring having 1 or 2 oxygen, sulfur or nitrogen atoms in above-described (2) and (3) may by substituted by 1 to 5 R^{5A} groups wherein multiple R^{5A}'s are independently a hydrogen atom, C1-6 alkyl, C1-6 alkoxy, C1-6 alkylthio, nitro, halogen, trifluoromethyl, trifluoromethoxy or hydroxy;
R^{2A} is CONR^{8A}, NR^{8A}CO, CONR^{8A}-C1-4 alkylene, C1-4 alkylene-CONR^{8A}, NR^{8A}CO-C1-4 alkylene, C1-4 alkylene-NR^{8A}CO, C1-3 alkylene-CONR^{8A}-C1-3 alkylene, C1-3 alkylene-NR^{8A}CO-C1-3 alkylene wherein R^{8A} is a hydrogen atom or C1-4 alkyl, O, S, NZ^{6A} wherein Z^{6A} is a hydrogen atom or C1-4 alkyl, Z^{7A}-C1-4 alkylene, C1-4 alkylene-Z^{7A}, C1-3 alkylene-Z^{7A}-C1-3 alkylene wherein Z^{7A} is O, S or NZ^{6A} wherein Z^{6A} has the same meaning as described above, CO, CO-C1-4 alkylene, C1-4 alkylene-CO, C1-3 alkylene-CO-C1-3 alkylene, C2-4 alkylene, C2-4 alkenylene or C2-4 alkynylene;
R^{3A} is a hydrogen atom, C1-6 alkyl, C1-6 alkoxy, C1-6 alkylthio, nitoro, halogen, trifluromethyl, trifluoromethoxy, hydroxy or hydroxymethyl;
R^{4A} is (1) a hydrogen atom, (2) C1-8 alkyl, C2-8 alkenyl, C2-8 alkynyl, (3) C1-6 alkyl substituted by 1 or 2 group(s) selected from COOZ^{8A}, CONZ^{9A}Z^{10A}, OZ^{8A} wherein Z^{8A}, Z^{9A} and Z^{10A} are independently a hydrogen atom or C1-4 alkyl, C1-4 alkoxy-C1-4 alkoxy, (4) C3-7 cycloalkyl, (5) C1-4 alkyl, C2-4 alkenyl or C2-4 alkynyl substituted by phenyl or C3-7 cycloalkyl wherein phenyl or C3-7 cycloalkyl in above-described (4) and (5) may be substituted by 1 to 5 R^{5A} groups wherein R^{5A} has the same meaning as described above; n^{A} and t^{A} are each independently an integer from 1 to 4, and
wherein
(1) R^{2A} and Z^{3A} are each connected at the 1- or 2-position of 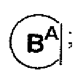 and
(2) when 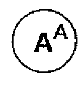 is benzene and (Z^{2A})_{1A} is other than COR^{1A}, Z^{1A} is connected at the 3- or 4-position of benzene,
a salt thereof, a solvate thereof or a prodrug thereof,
a compound represented by formula (B) 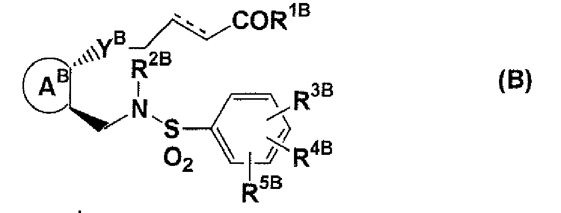 wherein 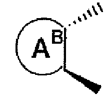 is a group represented by formula 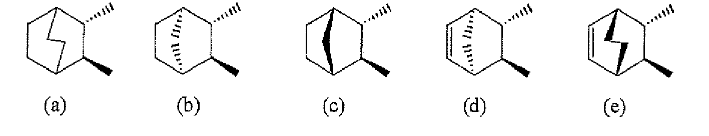 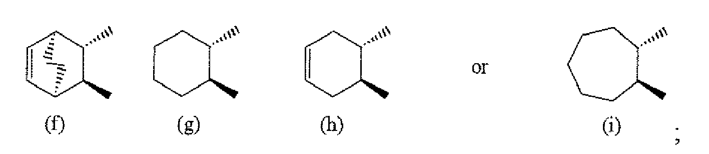 R^{1B} is hydroxy, C1-4 alkoxy or a group represented by formula NR^{6B}R^{7B} wherein R^{6B} and R^{7B} are each independently a hydrogen atom or C1-4 alkyl;
R^{2B} is a hydrogen atom or C1-4 alkyl;
R^{3B} and R^{4B} are each C1-4 alkyl, a halogen atom or trifluoromethyl;
R^{5B} is a hydrogen atom, C1-4 alkyl, a halogen atom or trifluoromethyl;
Y^{B} is cis-vinylene or trans-vinylene;
symbol 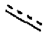 is a single bond or double bond, and
wherein, when 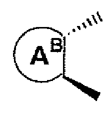 is formula 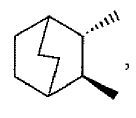 R^{1B} is hydroxy or C1-4 alkoxy, R^{2B} is a hydrogen atom, Y^{B} is cis-vinylene and symbol 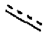 is a single bond, 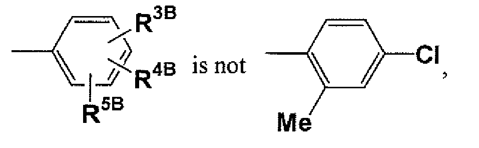 a salt thereof, a solvate thereof or a prodrug thereof, and
a compound represented by formula (C) 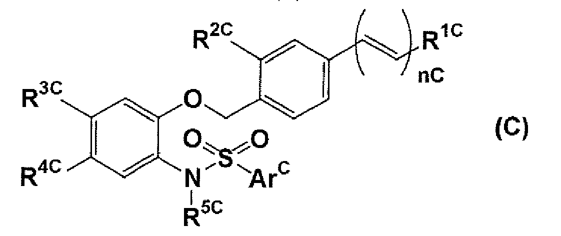 wherein R^{1C} is COOH, 5-tetrazolyl, 5-oxo-1,2,4-oxadiazolyl, CH₂OH or 5-oxo-1,2,4-thiadiazolyl;
R^{2C} is hydrogen, methyl, methoxy or chloro;
R^{3C} and R^{4C} are a combination of (1) methyl and methyl, (2) methyl and chloro, (3) chloro and methyl or (4) trifluoromethyl and hydrogen, or are taken together with the carbon atom to which they are attached to form (5) cyclopentene, (6) cyclohexene or (7) benzene;
R^{5C} is isopropyl, isobutyl, 2-methyl-2-propenyl, cyclopropylmethyl, methyl, ethyl, propyl, 2-propenyl or 2-hydroxy-2-methylpropyl;
Ar^{C} is thiazolyl which may be substituted by methyl, pyridyl or 5-methyl-2-furyl;
n^{C} is 0 or 1, and
wherein, when R^{1C} is 5-tetrazolyl, 5-oxo-1,2,4-oxadiazolyl or 5-oxo-1,2,4-thiadiazolyl, n^{C} is 0,
an alkyl ester thereof, a salt thereof or a prodrug thereof

12. The agent for preventing and/or treating urinary tract disease according to claim 11, wherein the compound is 4-[6-[N-isobutyl-N-(4-methyl-2-thiazolylsulfonyl)amino]indan-5-yloxymethyl]benzoic acid or 3-methyl-4-[6-[N-isobutyl-N-(4-methyl-2-thiazolylsulfonyl))amino]indan-5-yloxymethyl]cinnamic acid.

13. The agent for preventing and/or treating urinary tract disease according to claim 1, wherein the EP₃ antagonist is a compound selected from a group consisting of
a compound represented by formula (D) 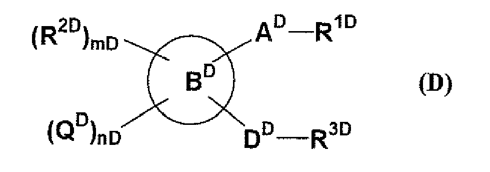 wherein R^{1D} is -COOH, -COOR^{4D}, -CH₂OH, -CONR^{5D}SO₂R^{6D}, -CONR^{7D}R^{8D}, -CH₂NR^{5D}SO₂R^{6D}, -CH₂NR^{9D}COR^{10D}, -CH₂NR^{9D}CONR^{5D}SO₂R^{6D}, -CH₂SO₂NR^{9D}COR^{10D}, -CH₂OCONR^{5D}SO₂R^{6D}, tetrazole, 1,2,4-oxadiazol-5-one, 1,2,4-oxadiazole-5-thione, 1,2,4-thiadiazol-5-one, 1,3-thiazolidine-2,4-dione, or 1,2,3,5-oxathiadiazol-2-one;
R^{4D} is C1-6 alkyl or (C1-4 alkylene)-R^{11D};
R^{11D} is hydroxy, C1-4 alkoxy, -COOH, C1-4 alkoxycarbonyl or -CONR^{7D}R^{8D};
R^{5D} is a hydrogen atom or C1-6 alkyl;
R^{6D} is
(i) C1-6 alkyl,
(ii) C3-15 mono-, bi- or tri-carbocyclic ring or 3- to 15-membered mono-, bi- or tri-heterocyclic ring substituted by 1 to 5 R^{12D} groups or unsubstituted, or
(iii) C1-6 alkyl, C2-6 alkenyl or C2-6 alkynyl substituted by C3-15 mono-, bi- or tri-carbocyclic ring or 3- to 15-membered mono-, bi- or tri-heterocyclic ring substituted by 1 to 5 R^{12D} groups or unsubstituted;
R^{7D} and R^{8D} are each independently
(i) a hydrogen atom,
(ii) C1-6 alkyl,
(iii) hydroxy,
(iv) -COR^{17D},
(v) C3-15 mono-, bi- or tri-carbocyclic ring or 3- to 15-membered mono-, bi- or tri-heterocyclic ring substituted by 1 to 5 R^{12D} groups or unsubstituted, or
(vi) C1-4 alkyl substituted by C3-15 mono-, bi- or tri-carbocyclic ring or 3- to 15-membered mono-, bi- or tri-heterocyclic ring substituted by 1 to 5 R^{12D} groups or unsubstituted;
R^{9D} is a hydrogen atom or C1-6 alkyl;
R^{10D} is
(i) a hydrogen atom,
(ii) C1-6 alkyl,
(iii) C3-15 mono-, bi- or tri-carbocyclic ring or 3-15 membered mono-, bi- or tri-heterocyclic ring substituted by 1 to 5 R^{12D} groups or unsubstituted, or
(iv) C1-6 alkyl, C2-6 alkenyl or C2-6 alkynyl substituted with C3-15 mono-, bi- or tri-carbocyclic ring or 3- to 15-membered mono-, bi- or tri-heterocyclic ring substituted by 1 to 5 R^{12D} groups or unsubstituted;
R^{12D} is (a) C1-6 alkyl, (b) C1-6 alkoxy, (c) C1-6 alkylthio, (d) a halogen atom, (e) CF₃, (f) cyano, (g) nitro, (h) hydroxy, (i) -COOR^{13D}, (j) -NHCOR^{13D}, (k) -SO₂R^{14D}, (l) -NR^{15D}R^{16D}, (m) C3-7 mono-carbocyclic ring substituted by C1-4 alkyl or oxo or unsubstituted, (n) 3- to 7-membered mono-heterocyclic ring substituted by C1-4 alkyl or oxo or unsubstituted or (o) C1-4 alkyl substituted by hydroxy, -COOR^{13D}, -NHCOR^{13D}, - SO₂R^{14D}, or -NR^{15D}R^{16D};
R^{13D} is a hydrogen atom, C1-4 alkyl, phenyl, or phenyl(C1-4)alkyl;
R^{14D} is C1-4 alkyl;
R^{15D} and R^{16D} are each independently a hydrogen atom, C1-4 alkyl, phenyl, phenyl(C1-4)alkyl;
R^{17D} is C1-4 alkyl or phenyl;
A^{D} is
(i) a single bond,
(ii) C1-6 alkylene,
(iii) C2-6 alkenylene,
(iv) C2-6 alkynylene,
(v) -O-(C1-3 alkylene),
(vi) -S-(C1-3 alkylene),
(vii) -NR^{20D}-(C1-3 alkylene),
(viii) -CONR^{21D}-(C1-3 alkylene),
(ix) -(C1-3 alkylene)-O-(C1-3 alkylene),
(x) -(C1-3 alkylene)-S-(C1-3 alkylene),
(xi) -(C1-3 alkylene)-NR^{20D}-(C1-3 alkylene),
(xii) -(C1-3 alkylene)-CONR^{21D}-(C1-3 alkylene),
(xiii) -Cyc1^{D},
(xiv) -(C1-4 alkylene)-Cyc1^{D}, or
(xv) -Cyc1^{D}-(C1-4 alkylene),
wherein the alkylene, alkenylene and alkynylene in A^{D} may be substituted by 1 to 6 substituents selected from the following substituents of (a)-(i):
(a) C1-6 alkyl, (b) C1-6 alkoxy, (c) halogen atom, (d) CHF₂, (e) CF₃, (f) OCHF₂, (g) OCF₃, (h) hydroxy, (i) hydroxy(C1-4) alkyl;
R^{20D} is a hydrogen atom, C1-4 alkyl, -SO₂(C1-4)alkyl or C2-5 acyl;
R^{21D} is a hydrogen atom or C1-4 alkyl;
Cyc1^{D} is C3-7 mono-carbocyclic ring or 3- to 7-membered mono-heterocyclic ring substituted with 1 to 4 substituents selected from C1-6 alkyl, C1-6 alkoxy, C1-6 alkylthio, C2-6 alkenyl, C2-6 alkynyl, halogen atom, CHF₂, CF₃, nitro and cyano or unsubstituted;
B^{D} ring is C3-12 mono- or bi-carbocyclic ring or 3- to 12-membered mono- or bi-heterocyclic ring;
R^{2D} is C1-6 alkyl, C1-6 alkoxy, C1-6 alkylthio, C2-6 alkenyl, C2-6 alkynyl, halogen atom, CHF₂, CF₃, nitro, cyano, phenyl or oxo;
m^{D} is 0, 1 or 2,
wherein
when -D-R^{3D} binds to B^{D} ring at the ortho position based on -A^{D}-R^{1D}, then n^{D} is 1 or 2, and
when -D-R^{3D} binds to B^{D} ring at the non-ortho position based on -A^{D}-R^{1D}, then n^{D} is 0, 1 or 2;
Q^{D} is
(1) (i) -(C1-4 alkylene, C2-4 alkenylene or C2-4 alkynylene)-Cyc2^{D},
(ii) -(C1-4 alkylene)-Z^{D}-Cyc3^{D},
(iii) C1-4 alkyl substituted by substituent(s) selected from -NR^{24D}R^{25D}, -S(O)_{pD}R^{26D}, cyano, -NR^{23D}COR^{27D}, -NR^{23D}SO₂R^{28D} and -NR^{23D}CONR^{24D}R^{25D}
(iv) a group selected from C1-4 alkoxy(C1-4)alkoxy, -NR^{23D}COR^{27D}, -COR^{28D}, -OSO₂R^{28D}, -NR^{23D}SO₂R^{28D} and -NR^{23D}CONR^{24D}R^{25D},
(v) C3-7 mono-carbocyclic ring or 3- to 6-membered mono-heterocyclic ring substituted with 1 to 5 R^{30D}'s, wherein one of the R^{30D}'s binds to the ring at the non 1-position,
(vi) C8-15 mono-, bi- or tri-carbocyclic ring or 7- to 15-membered mono-, bi- or tri-heterocyclic ring substituted by 1 to 5 R^{30D}'s or unsubstituted,
(vii) -T^{D}-Cyc5^{D} or
(viii) a group selected from -L^{D}-Cyc6-1^{D}, -L^{D}-(C3-6 cycloalkyl), -L^{D}-CH₂-(C3-6 cycloalkyl), -L^{D}-(C2-4 alkylene)-Cyc6^{D}-2 and -L^{D}-(C1-4 alkylene)_{qD}-Cyc6^{D}-3 wherein the C3-6 cycloalkyl is substituted by 1 to 5 R^{30D}'s or unsubstituted,
(2) (i) phenoxy,
(ii) benzyloxy,
(iii) hydroxy(C1-4)alkyl,
(iv) C1-4 alkoxy(C1-4)alkyl or
(v) -(C1-4 alkylene)-O-benzyl, or
(3) (i) C2-6 alkenyl,
(ii) C2-6 alkynyl,
(iii) C1-6 alkyl substituted by 1 to 3 halogen atoms,
(iv) cyano,
(v) nitro,
(vi) -NR^{33D}R^{34D},
(vii) -CONR^{33D}R^{34D},
(viii) -S(O)_{pD}-(C1-4)alkynyl,
(ix) -S(O)_{pD}-CHF₂,
(x) -S(O)_{pD}-NR^{33D}R^{34D},
(xi) -O-(C3-6)alkynyl,
(xii) -O-CHF₂, or
(xiii) C3-7 cycloalkyl;
R^{22D} is a hydrogen atom, C1-4 alkyl, -SO₂-(C1-4)alkyl or C2-5 acyl;
R^{23D} is a hydrogen atom, C1-4 alkyl, phenyl or phenyl(C1-4)alkyl;
R^{24D} and R^{25D} are each independently a hydrogen atom, C1-4 alkyl, Cyc4^{D} or (C1-4 alkylene)-Cyc4^{D};
R^{26D} is C1-4 alkyl or Cyc4^{D};
R^{27D} is a hydrogen atom, C1-4 alkyl, -OR^{29D} or Cyc4^{D};
R^{28D} is C1-4 alkyl, Cyc4^{D} or -(C1-4 alkylene)-Cyc4^{D};
R^{29D} is a hydrogen atom, C1-4 alkyl, Cyc4^{D} or (C1-4 alkylene)-Cyc4^{D};
R^{30D} is C1-8 alkyl, C1-8 alkoxy, C1-8 alkylthio, a halogen atom, CF₃, OCF₃, SCF₃, CHF₂, OCHF₂, SCHF₂, hydroxy, cyano, nitro, -NR^{31D}R^{32D}, -CONR^{31D}R^{32D}, formyl, C2-5 acyl, hydroxy(C1-4)alkyl, C1-4 alkoxy(C1-4)alkyl, C1-4 alkylthio(C1-4)alkyl, -(C1-4 alkylene)-CONR^{31D}R^{32D}, -SO₂(C1-4)alkyl, -NR^{23D}CO-(C1-4)alkyl,
-NR^{23D}SO₂-(C1-4)alkyl, benzoyl, oxo, C3-7 mono-carbocyclic ring, 3- to 7-membered mono-heterocyclic ring, -(C1-4 alkylene)-NR^{31D}R^{32D}, -M^{D}-(C3-7 mono-carbocyclic ring) or -M^{D}-(3- to 7-membered mono-heterocyclic ring),
wherein the C3-7 mono-carbocyclic ring and 3- to 7-membered mono-heterocyclic ring in R^{30D} may be substituted with 1 to 5 substituents selected from the following (a)-(1):
(a) C1-6 alkyl, (b) C2-6 alkenyl, (c) C2-6 alkynyl, (d) C1-6 alkoxy, (e) C1-6 alkylthio, (f) halogen atom, (g) CHF₂, (h) CF₃, (i) nitro, (j) cyano, (k) hydroxy, (1) amino;
M^{D} is -O-, -S-, C1-4 alkylene, -O-(C1-4 alkylene)-, -S-(C1-4 alkylene)-, -(C1-4 alkylene)-O-, or -(C1-4 alkylene)-S-;
R^{31D} and R^{32D} are each independently a hydrogen atom or C1-4 alkyl;
Cyc2^{D} is C3-15 mono-, bi- or tri-carbocyclic ring or 3- to 15-membered mono-, bi- or tri-heterocyclic ring substituted by 1 to 5 R^{30D}'s or unsubstituted;
Z^{D} is -O-, -S(O)_{pD}-, -NR^{22D}-, -NR^{23D}CO-, -NR^{23D}SO₂-, -NR^{22D}-(C1-4 alkylene)-, -S(O)_{pD}-(C1-4 alkylene)-, -O-(C2-4 alkylene)-, -NR^{23D}CO-(C1-4 alkylene) or -NR^{23D}SO₂₋(C1-4 alkylene);
p^{D} is 0, 1 or 2;
Cyc3^{D} is C3-15 mono-, bi- or tri-carbocyclic ring or 3- to 15-membered mono-, bi- or tri-heterocyclic ring substituted by 1 to 5 R^{30D}'s or unsubstituted;
Cyc4^{D} is C3-12 mono- or bi-carbocyclic ring or 3- to 12-membered mono- or bi-heterocyclic ring substituted by 1 to 5 R^{30D}'s or unsubstituted;
T^{D} is -O-, -NR^{22D}-, -O-(C1-4 alkylene)-, -S(O)_{pD}-(C1-4 alkylene)- or -NR^{22D}-(C1-4 alkylene);
Cyc5^{D} is 3- to 15-membered mono-, bi- or tri-heterocyclic ring substituted by 1 to 5 R^{30D}'s or unsubstituted;
q^{D} is 0 or 1;
L^{D} is -O- or -NR^{23D}-;
Cyc6-1^{D} is phenyl or benzyl substituted by one or more R^{30D}'s;
Cyc6-2^{D} is C3-6 mono-carbocyclic ring substituted by 1 to 5 R^{30D}'s or unsubstituted;
Cyc6-3^{D} is C7-15 mono-, bi- or tri-carbocyclic ring substituted by 1 to 5 R^{30D}'s or unsubstituted;
R^{33D} and R^{34D} are each independently a hydrogen atom, C1-4 alkyl, phenyl or benzyl, or
NR^{33D}R^{34D} representing 3- to 6-membered mono-heterocyclic ring which may contain one nitrogen atom and optional one hetero atom selected from nitrogen, oxygen and sulfur atom;
D^{D} is
(1) 1- or 2-membered linker comprising atom(s) selected from carbon, nitrogen, oxygen and sulfur atom, which may contain a double bond or a triple bond and may be substituted by 1 to 4 R^{40D}'s,
(2) 3- to 6-membered linker comprising atoms selected from carbon, nitrogen, oxygen and sulfur, which may contain double bond(s) or triple bond(s) and may be substituted by 1 to 12 R^{40D}'s, wherein R^{40D} substituted on the atom bound to R^{3D}, and R^{42D} which is a substituent of R^{3D} may be taken together to form -(CH₂)_{yD}- wherein y^{D} is 1 to 4, or
(3) 7- to 10-membered linker comprising atoms selected from carbon, nitrogen, oxygen and sulfur atom, which may contain double bonds or triple bonds and may be substituted by I to 20 R^{40D}'s, wherein R^{40D} substituted on the atom binding to R^{3D}, and R^{42D} which is a substituent of R^{3D} may be taken together to form -(CH₂)_{yD}-;
R^{40D} is (a) C1-8 alkyl, (b) C2-8 alkenyl, (c) C2-8 alkynyl, (d) oxo, (e) halogen atom, (f) CF₃, (g) hydroxy, (h) C1-6 alkoxy, (i) C2-6 alkenyloxy, (j) C2-6 alkynyloxy, (k) OCF₃, (l) -S(O)_{pD}-(C1-6)alkyl, (m) -S(O)_{pD}-(C2-6)alkenyl, (n) -S(O)_{pD}-(C2-6)alkynyl, (o) C2-5 acyl, (p) Cyc9^{D}, (q) C1-4 alkoxy(C1-4)alkoxy, (r) C1-8 alkyl, C2-8 alkenyl or C2-8 alkynyl substituted by 1 or 2 substituents selected from halogen atom, CF₃, OCF₃, hydroxy, cyano, C1-4 alkoxy, -S(O)_{pD}-(C1-6)alkyl, Cyc9^{D} and C1-4 alkoxy(C1-4)alkoxy, or
two R^{40D}'s may be taken together with the atom of a linker to which they bind to form C3-15 mono-, bi- or tri-carbocyclic ring or 3- to 15-membered mono-, bi- or tri-heterocyclic ring containing 1 to 2 hetero atoms selected from O, S, SO₂ and N, wherein the carbocyclic ring and the heterocyclic ring may be substituted by 1 to 3 substituents selected from C1-4 alkyl, C1-4 alkoxy, C2-5 acyl, SO₂(C1-4 alkyl), phenyl and phenyl(C1-4) alkyl;
Cyc9^{D} is C3-6 mono-carbocyclic ring or 3- to 6-membered mono-heterocyclic ring substituted by 1 to 5 R^{41D}'s or unsubstituted;
R^{41D} is C1-4 alkyl, C1-4 alkoxy, C1-4 alkylthio, C1-4 alkoxy(C1-4)alkyl, a halogen atom, CF₃, OCF₃, SCF₃, hydroxy, cyano, formyl, C2-5 acyl, -SO₂-(C1-4)alkyl, - NR^{23D}CO-(C1-4)alkyl, benzyl or oxo;
R^{3D} is
(1) C1-6 alkyl or
(2) C3-15 mono-, bi- or tri-carbocyclic ring or 3- to 15-membered mono-, bi- or tri-heterocyclic ring substituted by 1 to 5 R^{42D}'s or unsubstituted;
R^{42D} is (a) C1-6 alkyl, (b) C1-6 alkoxy, (c) C1-6 alkylthio, (d) a halogen atom, (e) cyano, (f) CF₃, (g) CHF₂, (h) OCF₃, (i) OCHF₂, (j) SCF₃, (k) -NR^{43D}R^{44D}, (l) -SO₂R^{45D}, (m) -NR^{46D}COR^{47D}, (n) hydroxy, (o) oxo, (p) C1-4 alkoxy(C1-4)alkyl, (q) Cyc10^{D}, (r) C1-6 alkylene-Cyc10^{D}, (s) -CO-Cyc10^{D}, (t) -W^{D}-Cyc10^{D}, (u) -(C1-6 alkylene)-W^{D}-Cyc10^{D}, (v) -W^{D}-(C1-6 alkylene)-Cyc10^{D} or (w) -(C1-6 alkylene)-W^{D}-(C1-6 alkylene)-Cyc10^{D};
R^{43D} and R^{44D} are each independently a hydrogen atom or C1-4 alkyl;
R^{45D} is C1-4 alkyl;
R^{46D} is a hydrogen atom or C1-4 alkyl;
R^{47D} is a hydrogen atom or C1-4 alkyl;
Cyc10^{D} is C3-12 mono- or bi-carbocyclic ring or 3- to 12-membered mono- or bi-heterocyclic ring substituted by 1 to 5 substituents selected from the following (a)-(j) or unsubstituted:
(a) C1-4 alkyl, (b) C2-5 acyl, (c) C1-4 alkoxy, (d) a halogen atom, (e) hydroxy, (f) nitro, (g) cyano, (h) amine, (i) CF₃, (j) OCF₃;
W^{D} is -O-, -S(O)_{pD}- or -NR^{48D}-;
R^{48D} is a hydrogen atom or C1-4 alkyl, a salt thereof, a solvate thereof or a prodrug thereof, and
a compound represented by formula (E) 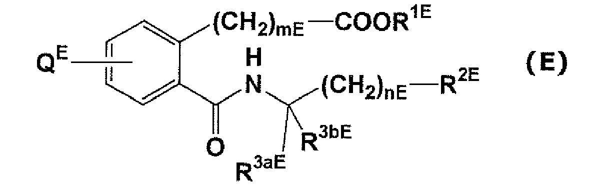 wherein R^{1E} is a hydrogen atom or C1-4 alkyl;
R^{2E} is phenyl, naphthyl, benzofuranyl or benzothienyl substituted by 1 or 2 substituents selected from C1-4 alkyl or a halogen atom or unsubstituted;
Q^{E} is (i) -CH₂-O-Cycl^{E}, (ii) -CH₂-Cyc2^{E} or (iii) -L-Cyc3;
Cyc1^{E} is phenyl or pyridyl substituted by one or two R^{4E}'s or unsubstituted;
Cyc2^{E} is indolyl substituted by one or two R^{4E}'s or unsubstituted;
Cyc3^{E} is phenyl substituted by one or two R^{4E}'s or unsubstituted;
L is -O- or -NH-;
R^{3a} and R^{3b} are each independently a hydrogen atom or C1-4 alkyl, or are taken together with the carbon atom to which R^{3aE} and R^{3bE} are attached to form tetrahydro-2H-pyran;
mE is 2 or 3;
nE is 0, 1 or 2;
R^{4E} is C1-4 alkyl, C1-4 alkylthio, a halogen atom or cyano, or when Cyc3^{E} is phenyl substituted by two R^{4E}'s, and two R^{4E}'s, together with phenyl, may form 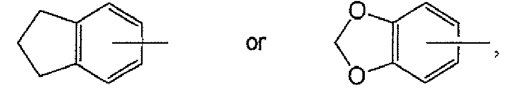 a salt thereof, a solvate thereof or a prodrug thereof.

14. The agent for preventing and/or treating urinary tract disease according to claim 13, wherein the compound is N-(3,4-difluorophenylsulfonyl)-3-(2-(2-(naphthalen-2-yl)ethoxy)-4-(3-cyanophenoxymethyl)phenyl)propanamide, 3-[4-[(2,5-dimethylphenoxy)methyl]-2-({[(1R)-1-(3,5-diethylphenyl)-3-methylbutyl]amino}carbonyl)phenyl]propanoic acid, 3-(2-(((1R)-3-methyl-1-(3,5-dimethylphenyl)butyl)carbamoyl)-4-(2,5-difluorophenoxymethyl)phenyl)propanoic acid, or 3-(2-((((1R)-1-(3,5-dimethylphenyl)-3-methylbutyl)amino)carbonyl)-4-(5-fluoro-2-methylphenoxymethyl)phenyl)propanoic acid.

15. The agent for preventing and/or treating urinary tract disease according to claim 1, wherein the EP₁ antagonist and the EP₃ antagonist are used at low doses.

16. An agent for preventing and/or treating urinary tract disease, which comprises a compound having antagonism to EP₁ and antagonism to EP₃.

17. A method for preventing and/or treating urinary tract disease, which comprises administering an effective amount of a medicament comprising a combination of EP₁ antagonist and EP₃ antagonist to a mammal.

18. A method for preventing and/or treating urinary tract disease, which comprises administering an effective amount of a compound having antagonism to EP₁ and antagonism to EP₃ to a mammal.

19. Use of a combination of EP₁ antagonist and EP₃ antagonist, for the preparation of an agent for preventing and/or treating urinary tract disease.

20. Use of a compound having antagonism to EP₁ and antagonism to EP₃, for the preparation of an agent for preventing and/or treating urinary tract disease.
